# EUROPÄISCHE PATENTANMELDUNG

(11) **EP 1 088 885 A2**
(43) Veröffentlichungstag der Anmeldung: **04.04.2001**
(21) Anmeldenummer: 00121209.1
(22) Anmeldetag: 29.09.2000
(51) Int. Cl.: C12M 1/107

(54) **Verfahren und Reaktor zur Erzeugung von Biogas**

(30) Priorität: 01.10.1999 DE 19947339; 01.10.1999 DE 19947340
(71) Anmelder: Tentscher, Wolfgang, Dr., 12167 Berlin (DE)
(72) Erfinder: Tentscher, Wolfgang, Dr., 12167 Berlin (DE)
(74) Vertreter: Schwabe - Sandmair - Marx

(57) **Zusammenfassung**

Ein Biogasreaktor umfasst:
a) wenigstens einen Reaktorbehälter (T4a, T4b) zur Erzeugung eines Biogases durch fermentativen Abbau einer Biomasse,
b) und einen Seitenschacht (T3) für eine Einbringung von Biomasse in den Reaktorbehälter (T4a, T4b),
c) wobei der Seitenschacht (T3) eine untere Öffnung (F13), durch welche Biomasse in dem fermentativen Abbau förderbar ist, und aus dem Seitenschacht (T3) eine obere Öffnung (F12) aufweist, durch welche ausgefaulte Flüssigkeit aus dem Abbau in den Seitenschacht (T3) nachströmen kann.
   Der Reaktor zeichnet sich dadurch aus, dass
d) der Seitenschacht (T3) außen an oder neben dem Reaktorbehälter (T4a, T4b) angeordnet und über die obere Öffnung (F12) und die untere Öffnung (F13) mit dem Reaktorbehälter (T4a, T4b) verbunden oder verbindbar ist.

## Beschreibung

Die Erfindung betrifft ein Verfahren und einen Reaktor zur Erzeugung von Biogas.

In der Erzeugung von Biogas in Biogasreaktoren für einen nicht-sterilen fermentativen Abbau einer Biomasse kommt der guten Durchmischung der Biomasse im Reaktor eine große Bedeutung zu. Probleme bereitet oft die Zuführung von frischer Biomasse, insbesondere wenn die frische Biomasse zu einem größeren Teil aus Fasermaterial, beispielsweise ganzen Pflanzen, Teilen von Pflanzen, Silage und dergleichen besteht. Bekannt ist die Zuführung der frischen Biomasse in den Fermentationsprozess durch einen Seitenschacht, der unmittelbar an einer Behälterwand des Reaktors oder zumindest in räumlicher Nähe des Reaktors angeordnet ist.

Ein Biogasreaktor mit einem äußeren Einwurfschacht ist aus der DE 299 02 042 U1 bekannt. Die WO 99/32600 beschreibt einen Biogasreaktor mit einem Seitenschacht, der unmittelbar an einer Seitenwand im Inneren des Reaktors angeordnet ist. Eine obere Öffnung des Seitenschachts liegt unterhalb der freien Oberfläche der ausgefaulten Flüssigkeit in dem Reaktor. Eine Pumpe zur Durchmischung der Biomasse in dem Reaktor ist nahe bei einer unteren Öffnung des Seitenschachts angeordnet, über die der Seitenschacht mit dem Behälterinnenraum des Reaktors verbunden ist. Frische Biomasse wird durch einen äußeren Stutzen über die Seitenwand des Reaktorbehälters oberhalb der oberen Öffnung des Seitenschachts zugeführt. Die Zuführung von Pflanzenteilen oder gar ganzen Pflanzen oder auch grundsätzlich von faserigem Material ist schwierig, wenn nicht gar unmöglich. Auch die Einführung der frischen Biomasse unmittelbar in die aus gefaulte Flüssigkeit in dem Reaktor wirft Probleme auf.

Eine Aufgabe der Erfindung ist es, die Erzeugung von Biogas und den Betrieb eines Biogasreaktors zu verbessern.

Es wird Biogas aus einer Biomasse erzeugt, die vorzugsweise eine Mischung ist aus Gülle und/oder organischen Abfällen und separat angebauten Pflanzen, d.h. nachwachsenden Rohstoffen. Dies geschieht in einem nicht sterilen fermentativen, vorzugsweise anaeroben, Abbau mittels Mikroorganismen. Ein bei dem Abbau entstehendes Biogasgemisch wird vorzugsweise zu einem erdgasgleichen Biomethan und Kohlendioxid aufbereitet. Da als nachwachsender Rohstoff für die Erfindung vorzugsweise Pflanzen verwendet werden, wie sie in anderen Bereichen der Technik auch zur Energieerzeugung eingesetzt werden, werden die Pflanzen dieser Biofraktion nachfolgend als Energiepflanzen bezeichnet.

Bisher wird Gülle hingegen allein oder in Verbindung mit Abfällen ausgefault und ein dabei entstehendes Biogas in Blockheizkraftwerken verstromt. Die Abgase werden in die Atmosphäre emittiert. Die BHKW-Abwärme ist nur zu einem Teil nutzbar. Der Wärmeüberschuss, der totgekühlt werden muss, ist besonders in der Sommerzeit sehr hoch.

Die Zufuhr von frischer Biomasse erfolgt über einen Seitenschacht, der außerhalb des Reaktors in räumlicher Nähe des Reaktors oder vorzugsweise unmittelbar an einer Reaktorseitenwand stehend angeordnet ist. Die Anordnung außerhalb des Reaktors hat den Vorteil, dass die frische Biomasse sehr bequem durch eine obere Öffnung, vorzugsweise eine Öffnung in der oberen Stirnseite des Seitenschachts, eingefüllt werden kann. Frische Biomasse kann vorteilhafterweise durch diese obere Einfüllöffnung hindurch eingeworfen werden. Der Seitenschacht ist teilweise mit Flüssigkeit gefüllt, mit der zusammen die frische Biomasse durch eine untere Öffnung des Seitenschachts, die mit einem unteren Teil des Reaktorbehälters verbunden ist, in den Reaktorbehälter gefördert wird. Die Förderung erfolgt vorzugsweise mittels einer Fördereinrichtung, die noch im Seitenschacht bei der unteren Öffnung oder in einem Verbindungskanal, der von der unteren Öffnung des Seitenschachts in das Reaktorinnere führt, angeordnet ist.

Indem erfindungsgemäß der Seitenschacht über die genannte obere Öffnung oder eine weitere obere Öffnung mit der ausgefaulten Flüssigkeit in dem Reaktor verbunden oder verbindbar ist, kann die ausgefaulte Flüssigkeit aus dem Reaktor auf die in den Seitenschacht eingebrachte frische Biomasse geleitet werden. Die Einleitung der ausgefaulten Flüssigkeit von oben auf die frische Biomasse ist insbesondere zum Einbringen von leichter Biomasse von Vorteil, die auf der Flüssigkeit im Seitenschacht sonst nur aufschwimmen würde. Die Einleitung der ausgefaulten Flüssigkeit bringt einen zusätzlichen Durchmischungs- und Spüleffekt, der die Förderung in den Reaktorbehälter unterstützt.

Die Einleitung der ausgefaulten Flüssigkeit in den Seitenschacht kann durch aktive Förderung, beispielsweise mittels einer Pumpe erhalten werden. Vorzugsweise ist die obere Öffnung zum Einleiten der ausgefaulten Flüssigkeit auf solch einer Höhe vorgesehen, dass die ausgefaulte Flüssigkeit aufgrund ihres eigenen hydrostatischen Drucks in den Seitenschacht strömt. Die obere Öffnung ist im Seitenschacht ferner vorteilhafterweise so angeordnet, dass sie über einer Flüssigkeitssäule in dem Seitenschacht liegt. Besonders vorteilhaft ist es, wenn ein Vormisch- und Spüleffekt im Seitenschacht durch die kombinierte Wirkung einer Fördereinrichtung und der nachströmenden Flüssigkeit erfolgt. Durch eine Fördereinrichtung, mit welcher das Gemisch aus Flüssigkeit und frischer Biomasse durch die untere Öffnung des Seitenschachts gegen den hydrostatischen Druck der Flüssigkeitssäule im Reaktorbehälter abgepumpt bzw. aus dem Seitenschacht abgesaugt werden kann, ist zudem auch die Einstellung der Flüssigkeitssäule in dem Seitenschacht auch auf eine vorteilhafte, grundsätzlich aber beliebige Höhe möglich.

Eine bevorzugte Biogasanlage weist eine Gastrennungseinrichtung auf, die dem Biogasreaktor nachgeschaltet ist. Die Gastrennungseinrichtung trennt erdgasgleiches Biomethan und Kohlendioxid aus dem erzeugten Biogas. Die Anlage oder nur der Reaktor eignet sich insbesondere zu Aufstellungen in einer landwirtschaftlichen Umgebung. Falls das erzeugte Biogas nicht ausreichend rein an Biomethan und Kohlendioxid ist, entsteht ein Restgasstrom, der anderweitig genutzt oder nur entsorgt, beispielsweise einfach abgelassen wird.

Ein Vorteil des bevorzugt abgebauten Gemisches besteht darin, dass den bereits heute in Biogasanlagen abgebauten Biomassen Gülle und/oder organischer Abfall ein nachwachsender Rohstoff in Form der separat angebauten Energiepflanzen beigegeben wird und dadurch eine Biogasanlage mit einem stets optimalen Auslastungsgrad betrieben werden kann. Bei den organischen Abfällen handelt es sich insbesondere um organische Haushaltsabfälle, wie sie in Deutschland beispielsweise in der sogenannten Biotonne gesammelt werden, und organische Abfälle der Industrie. Bevorzugt wird Gülle aus der Tierhaltung verwertet. Durch die gleichzeitige Vergärung von Pflanzen mit Gülle und/oder organischen Abfällen ist es ferner möglich, größere Biogasanlagen zu bauen, die vorzugsweise in einem Leistungsbereich von 0,5 bis 5 MW arbeiten und die eine wirtschaftliche Verwertung von Gülle auch aus kleinen Tierhaltungen durch den economy-of-scale-effekt ermöglichen. Gülle wird bisher nur zu weniger als 1 % für die Biogaserzeugung genutzt.

Mit den beiden bevorzugten Endprodukten, nämlich erdgasgleiches Biomethan und Kohlendioxid, liefert die Erfindung Produkte, die sich für eine Verwertung durch die Allgemeinheit unmittelbar eignen. Das erzeugte Biomethan kann insbesondere als Energielieferant dienen. Durch den Anbau und Zumischung von leicht vergärbarer Biomasse kann aus Gülle und/oder Abfällen auch zusammen mit anderweitig gewonnenem Methan ein Teil des Erdgasbedarfes durch erneuerbares Biomethan ersetzt werden. Das weitere Endprodukt Kohlendioxid steht für jede für diesen Rohstoff bekannte Verwertung zur Verfügung.

Besonders bevorzugt wird das erdgasgleiche Biomethan in ein Leitungsnetz eingeleitet. Hierbei kann es sich um ein bestehendes Erdgasnetz handeln. Es kann jedoch auch ein eigenes, zumindest in einer ersten Ausbaustufe lokal begrenzten Netz für die Einleitung und Durchleitung des Biomethans zu interessierten Verbrauchern, insbesondere zu Industriebetrieben, erst errichtet werden. Ein Vorteil liegt darin, dass das Biomethan anderweitig erzeugtem Erdgas, das bereits heute durch stationäre Leitungsnetze gefördert wird, zugemischt und mit diesem Erdgas transportiert werden kann. Es kann stattdessen oder auch zusätzlich in einer lokalen Anlage auf 250 bar komprimiert und in Druckbehälter abgefüllt werden.

Das abgetrennte Kohlendioxid ist vorzugsweise so rein, dass es einer direkten wirtschaftlichen Verwertung zuführbar ist, die beispielsweise in der Landwirtschaft in der näheren Umgebung der Biogasanlage erfolgen kann. Durch die direkte Verwertung von Kohlendioxid braucht der lange Kohlenstoffkreislauf über die Atmosphäre nicht abgewartet zu werden, bei dem bis zu 100 Jahre vergehen können, bis emittierter Kohlenstoff wieder assimiliert wird. Erst nach der wirtschaftlichen Verwertung des erfindungsgemäß erzeugten Kohlendioxids wird der Kohlenstoffkreislauf der Natur geschlossen. Bevorzugt wird das Kohlendioxid in ein bestehendes oder extra errichtetes CO₂-Leitungsnetz eingespeist, das bis zur Anlage verlegt ist.

Dank einer in anderen Verwendungen altbekannten und bewährten feuchten Konservierungsmethode, der Silierung, kann die eigens für den fermentativen Abbau angebaute Biomasse über etwa 10 Monate hinweg in einem Silo konserviert werden, so dass der Reaktor gleichmäßig und täglich mit einer Mischung von Silage und Gülle und/oder organischen Abfällen beschickt werden kann. Das Silo befindet sich vorzugsweise am Ort der Biogasanlage, kann grundsätzlich aber auch von ihr entfernt gelegen sein. Das Silo ist mit Leitungen zur Einleitung von Kohlendioxid auf eine kompaktierte Pflanzenmasse und mit Leitungen am Siloboden ausgestattet. Die Speicherung mittels Silierung hat den Vorteil, dass die Kapazität des Reaktors oder der Biogasanlage an den Erdgasbedarf angepasst werden kann, der in der Winterzeit viel höher als in der Sommerzeit ist. Die Kapazitätsauslegung der Biogasanlage erfolgt deshalb vorzugsweise für den Winterbedarf. Die Ressourcenproduktivität des Faktors Boden, d.h. der Energieertrag pro Fläche bei einer Biogaserzeugung aus Ganzpflanzen, lässt sich gegenüber der von RME (Rapsmethylester) um das Drei- bis Vierfache erhöhen. Zudem hat Biogas sehr geringe CO₂-Minderungskosten in Höhe von etwa DM 200/t CO₂, zu heutigen Preisen. Bei RME sind CO₂-Minderungskosten in Höhe von etwa DM 1.450/t CO₂ bekannt. Vorzugsweise werden gehäckselte Ganzpflanzen nach der Ernte in einem Fahrsilo siliert und zwischengespeichert.

Die in dem Biogasreaktor abzubauende Mischung wird vorzugsweise in einer Vormischeinrichtung, beispielsweise in einer Vorgrube am Ort der Anlage oder unmittelbar in dem Seitenschacht gemischt und homogenisiert. Besonders bevorzugt wird die Mischung in einem aus mehreren Teilreaktoren bzw. Behältern bestehenden Biogasreaktor aus gefault.

Ein rohes Biogas und ein entschwefeltes Biogas werden separat in je wenigstens einem Gasspeicher, vorzugsweise in je wenigstens einem Niederdruckspeicher, zwischengespeichert. Die Speicherung erfolgt bevorzugt ohne Luft- bzw. Sauerstoffzugabe. Besonders bevorzugt erfolgt sie unter Luft- bzw. Sauerstoffabschluss.

Der Biogasreaktor ist über eine Gasleitung vorzugsweise direkt mit dem Gasspeicher für rohes Biogas verbunden. Rohes Biogas wird aus dem Gasspeicher in die Gastrennungseinrichtung gefördert. Vorzugsweise wird der Speicher für rohes Biogas von einer Membran gasdicht nach oben abgeschlossen. Die Membran ist luftundurchlässig. Vorzugsweise weist sie mehrere übereinanderliegende Schichten auf. Die Gaspermeabilität jeder der Schichten beträgt bei 0 bis 30°C vorzugsweise höchstens 150 cm³/(m²bar24h). Besonders bevorzugt bildet die Membran einen Schutzraum. Der Abstand zwischen den Schichten der Membran, falls die Membran ein Gasspeichersack oder Gasspeicherkissen ist, wird mit Abstandshaltern konstant gehalten. Die Abstandshalter sind vorzugsweise so ausgebildet, dass sie einen kontrollierten Gasfluss unterstützen und Rückströmungen und Kurzschlussströmungen verhindern. Der Schutzraum hat vorzugsweise ein variables Volumen. Zur Einstellung eines variablen Volumens kann ein starrer Behälter verwendet werden, in dem die flexible Membran befestigt ist.

Vorzugsweise ist der Schutzraum mit rohem und/oder entschwefeltem Biogas und/oder einer Flüssigkeit gefüllt. Bei einer Befüllung mit Biogas kann das Biogas nach einem Durchströmen des Schutzraumes in eine Energiestation der Anlage gefördert und zur Energieerzeugung genutzt werden. Bei einer Befüllung des Schutzraums mit einer Flüssigkeit wird bereits durch die Flüssigkeit eine ausreichende Dichtigkeit gewährleistet. Die Dichtigkeitsanforderungen an die Membranen können verringert werden. Eine Membran, die den Gasraum im Gasspeicher von der Flüssigkeit trennt, kann sogar semipermeabel sein. Durch die Membran dringendes Biogas wird von der Flüssigkeit aufgenommen. Die Flüssigkeit kann den Schutzraum langsam durchströmen oder batchweise ausgetauscht werden. Vorzugsweise bildet Wasser die Flüssigkeit. Dies bringt den Vorteil der einfachen Entsorgbarkeit mit sich. Vorzugsweise wird das Wasser mit darin aufgenommenem Biogas in den Reaktor zurückgeführt.

Vorzugsweise ein Teil des Biogases wird in einer Energiestation zur Energiebedarfsdeckung der Biogasanlage an Wärme und Kälte und/oder Strom genutzt bzw. umgewandelt.

Eine vorzugsweise durchgeführte Entschwefelung des Biogases erfolgt bevorzugt ohne Luftzusatz, insbesondere ohne Sauerstoffzugabe. Besonders bevorzugt erfolgt sie unter Abschluss von Luft bzw. Sauerstoff. Eine vollständige Entschwefelung des Biogasgemisches wird bis zu einem Restschwefelgehalt von 0,1 bis 30 mg/m³ in einer Gastrennungseinrichtung durchgeführt. Die Entschwefelung des Biogasgemisches kann mit Aktivkohle und/oder in Molekularsieben und/oder in gasangeströmten, hydrophoben, flüssigkeitsdurchströmten Membranen erfolgen.

In der Gastrennungseinrichtung wird ein vorzugsweise zuvor entschwefeltes Biogas in Methan und Kohlendioxid getrennt. Bevorzugt erfolgt die Entschwefelung in einer eigenen Entschwefelungsstufe und die Trennung von Biomethan und Kohlendioxid in einer anschließend mit dem entschwefelten Biogas durchgeführten Methan-CO₂-Trennstufe oder mehreren Methan-CO₂-Trennstufen der Gastrennung. Vorzugsweise sind die Entschwefelung und die Methan-CO₂-Trennung voneinander separiert, und es kann das entschwefelte Biogas der Methan-CO₂-Trennung und wahlweise dem Speicher für entschwefeltes Biogas für eine anderweitige Verwendung zugeführt werden.

In einer besonders bevorzugten Ausführungsform erfolgt die Trennung von Methan und CO₂ in mehreren, in Serie geschalteten Methan-CO₂-Trennstufen. Insbesondere erfolgt sie in zwei hintereinander geschalteten Methan-CO₂-Trennstufen. In einer ersten der mehreren Methan-CO₂-Trennstufe wird ein methanreicher Teilstrom und ein kohlendioxidreicher Teilstrom gebildet. Werden die Trennstufen als PSA-Kolonnen ausgebildet, welche mit Molekularsieben oder Flüssigkeit gefüllt sind, so fällt der kohlendioxidreiche Teilstrom als Desorptions- bzw. Evakuierungsgas an. Der kohlendioxidreiche Teilstrom wird einer zweiten, nachgeschalteten Methan-CO₂-Trennstufe zugeführt, vorzugsweise jedoch nur, falls der Methanrestgehalt in dem kohlendioxidreichen Teilstrom unterhalb eines vorgegebenen Werts liegt. Liegt der Methanrestgehalt im kohlendioxidreichen Teilstrom über dem vorgegebenen Wert, so wird der kohlendioxidreiche Teilstrom vorzugsweise in eine frühere Verfahrensstufe zurückgeführt. Besonders bevorzugt wird er in diesem Fall in den Reaktor in den fermentativen Abbau, in ein Organikvorfilter einer Gastrennungseinrichtung und/oder in die erste Metan-CO₂-Trennstufe zurückgeführt. Der genannte, vorgegebene Wert für den Restgehalt an Methan im kohlendioxidreichen Teilstrom liegt vorzugsweise bei 1 Vol. %. In der nachgeschalteten Methan-CO₂-Trennstufe erfolgt eine weitere Anreicherung des Kohlendioxids im kohlendioxidreichen Teilstrom, so dass vorzugsweise Kohlendioxid in einer für beliebige wirtschaftliche Anwendungen ausreichenden Reinheit erhalten wird.

Dementsprechend weist die Gastrennungseinrichtung eine erste Methan-CO₂-Trennstufe auf, die aus dem Biogas einen methanreichen Teilstrom und einen kohlendioxidreichen Teilstrom trennt, und wenigstens eine zweite Methan-CO₂-Trennstufe, wobei ein Auslaß der ersten Methan-CO₂-Trennstufe für den kohlendioxidreichen Teilstrom durch eine Fluidverbindung mit einem Einlass der zweiten Methan-CO₂-Trennstufe verbunden ist. In der Fluidverbindung ist vorzugsweise eine Verzweigung vorgesehen, durch die der kohlendioxidreiche Teilstrom der zweiten Methan-CO₂-Trennstufe zuführbar oder in wenigstens einen der zweiten Methan-CO₂-Trennstufe vorgeschalteten Teil der Anlage zurückführbar ist. Der vorgeschaltete Anlagenteil wird vorzugsweise durch einen Festbettreaktor gebildet, der mit einem Flüssigkeitsauslass des Biogasreaktors verbunden ist und in dem der zurückgeführte, kohlendioxidreiche Teilstrom durch einen Rieseistrom einer ausgefaulten Flüssigkeit aus dem Biogasreaktor geführt wird.

Der Wobbeindex des erdgasgleichen Biomethans ist vorzugsweise nach DVGW G 260 als erdgasgleiches Gas aufbereitet.

Das Kohlendioxid hat eine Reinheit von vorzugsweise über 99 %. Besonders bevorzugt wird Kohlendioxid mit Lebensmittelqualität gewonnen.

Die ausgefaulte Flüssigkeit wird in einem vorzugsweise mit einer Gasspeicherfolie abgedeckten Lagertank, der gleichzeitig Nachgärtank ist, für vorzugsweise 1 Monat und in vorzugsweise weiteren Lagertanks für vorzugsweise weitere 5 Monate zwischengespeichert und bevorzugt als Dünger auf landwirtschaftlichen Flächen verwendet.

Nachwachsende Rohstoffe werden vorzugsweise als ganze Pflanze geerntet und erfindungsgemäß zur Biogaserzeugung verwendet, im Vergleich zu Biodiesel, bei dem nur das Saatgut zur Energieerzeugung genutzt wird.

Vorzugsweise weist eine aus den Energiepflanzen bestehende Biomasse für den Abbau 20-60% Trockensubstanz auf. Vorteilhaft ist die Ausfaulung von frischen Pflanzen, angewelkten Pflanzen oder Silage oder einer Mischung daraus mit vorzugsweise 20-60% Trockensubstanz und Gülle mit vorzugsweise 4-20% Trockensubstanz in Biogasreaktoren.

Liegende vollständig gefüllte Reaktoren mit horizontalem Rührwerk haben sich als besonders robust erwiesen, um Mischungen mit hohen Trockensubstarizgehalten von vorzugsweise 10 bis 30% ohne Verstopfungen auszufaulen. Während der Ausfaulung werden die Trockensubstanz und die Viskosität bereits erheblich verringert. Stehende Gärbehälter haben sich im Nassfermentationsverfahren mit Trockengehalten von vorzugsweise 5 bis 15% bewährt. Liegende und stehende Gärbehälter können deshalb vorteilhaft miteinander in Reihenschaltung gekoppelt werden. Der liegende, vorzugsweise kleinere Gärtank wird als erste Stufe eingesetzt. Vorteilhaft ist auch die Zusammenschaltung stehender Reaktoren, vorzugsweise im Nassfermentationsverfahren.

Die Verwendung einer Mischung aus Energiepflanzen und wenigstens einer der weiteren Biomassen Gülle und organische Abfälle für den fermentativen Abbau und die anschließende Aufbereitung des derart erzeugten Biogases zu einem erdgasgleichen Methanprodukt und einem in Industrie und Landwirtschaft ohne weiteres verwendbaren Kohlendioxidprodukt ist auch an sich ohne die beanspruchte Erfindung vorteilhaft. Ebenso gilt dies für die Ausbildung eines oder mehrerer Biogasspeicher als luftundurchlässige Speicher und auch für die Entschwefelung von Biogas ohne Luft- bzw. Sauerstoffzugabe. Auch die Einleitung von aus einem Biogas hergestelltem Biomethan und/oder Kohlendioxid in ein fest verlegtes Leitungsnetz kann für sich allein oder in Kombination mit offenbarten Merkmalen zum Vorteil eingesetzt werden. Diesbezüglich wird die DE 199 47 340 vollinhaltlich, und insbesondere deren Anspruchsgegenstände, in Bezug genommen. Der Anmelder behält es sich insbesondere vor, für eine Teilungsanmeldung auf diese Anmeldung auch alleine zurückzugreifen. Die weiteren Erfindungen kommen zwar bevorzugt in Kombination mit der beanspruchten Erfindung zum Einsatz. Sie können vorteilhaft aber auch bei einer anderen Art der Biogaserzeugung eingesetzt werden. Schließlich ist auch ein Silo mit einem im Silo verlegten Leitungsnetz zur Einleitung von CO₂ in eine zu silierende Schüttung alleine für eine Silierung nutzbringend einsetzbar.

Bevorzugte Ausführungsbeispiele der Erfindung werden nachfolgend anhand von Figuren erläutert. Es zeigen:
- Fig. 1: ein Fahrsilo,
- Fig. 2: einen Biogasreaktor mit zwei Reaktorbehältern und einer separaten Vorgrube als Vormischeinrichtung,
- Fig. 3: einen Reaktorbehälter mit einem integrierten Seitenschacht als Vormischeinrichtung in einer ersten Ausführung,
- Fig. 4: einen Reaktorbehälter mit einem integrierten Seitenschacht als Vormischeinrichtung in einer zweiten Ausführung,
- Fig. 5a: den Seitenschacht der Figuren 3 und 4 in einem anderen Schnitt,
- Fig. 5b: einen Reaktorbehälter mit einem abgesetzten Seitenschacht in einer ersten Ausführung in einem Längsschnitt,
- Fig. 5c: den Reaktorbehälter mit Seitenschacht der Fig. 5b in einem Querschnitt,
- Fig. 5d: einen Reaktorbehälter mit abgesetztem Seitenschacht in einer zweiten Ausführung in einem Querschnitt,
- Fig. 6: eine Reihenschaltung eines liegenden und eines stehenden Reaktorbehälters,
- Fig. 7: eine Biogasspeicherung und Biogastrennung
- Fig. 8: den Speicher für entschwefeltes Biogas gemäß Fig. 6 und
- Fig. 9: eine Gastrennungseinrichtung.

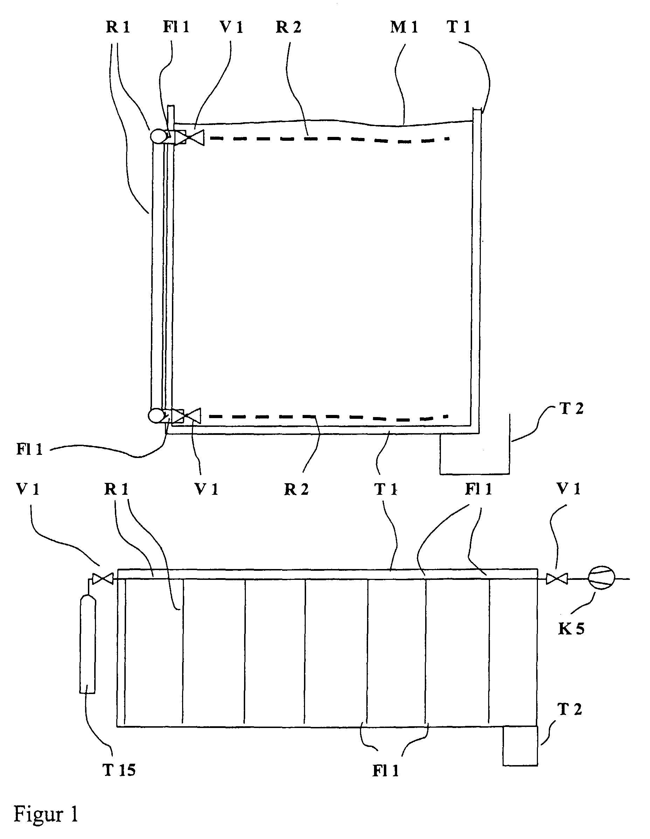

Figur 1 zeigt ein Fahrsilo T1 in einem Querschnitt und darunter in einer Ansicht. Energiepflanzen, vorzugsweise Silomais, Raps, Weizen, Roggen, Hirse, Luzerne, Futterrüben, Zuckerrüben, Kartoffeln und/oder Gräser, werden vorzugsweise als Ganzpflanzen mit Feldhäckslern gehäckselt, zum Fahrsilo T1 transportiert und dort einsiliert. Der Siliervorgang umfasst die Zugabe von Silierhilfsmitteln, wie beispielsweise Melasse und/oder Impfkulturen, vorzugsweise eine Kompaktierung der losen Schüttung auf eine Schüttdichte von vorzugsweise 300 bis 600 kg/m³ und Abdeckung mit Silofolie M1. Das Fahrsilo T1 ist mit einer waagerechten, vorzugsweise entlang des oberen Silorandes verlegten Rohrleitung R1 von vorzugsweise 1/2 bis 3 Zoll Durchmesser mit senkrechten, in vorzugsweise 2 bis 4 m Abstand angebrachten Abzweigungen zum Siloboden ausgestattet. Die senkrechten Abzweigungen haben Anschlüsse Fl 1 an den Enden. Von diesen Anschlüssen sind perforierte Leitungen, vorzugsweise Schläuche R2, am Siloboden und auf die kompaktierten Pflanzen verlegt, über die eine vorzugsweise dem 1 bis 3-fachen des Porenvolumens entsprechende Menge an Kohlendioxid über direkt angeschlossene Druckflaschen T 15 oder über ein Gebläse K5 zugegeben wird, um sowohl gleich nach Kompaktierung als auch nach Abdeckung mit der Folie M1 zu Beginn des Siliervorganges den Sauerstoff zu verdrängen und die Silierverluste zu verringern. Die am Boden verlegten Schläuche R2 liegen vorteilhafterweise in Aussparungen bzw. Vertiefungen. Mit T2 ist eine Sickerwassergrube bezeichnet.

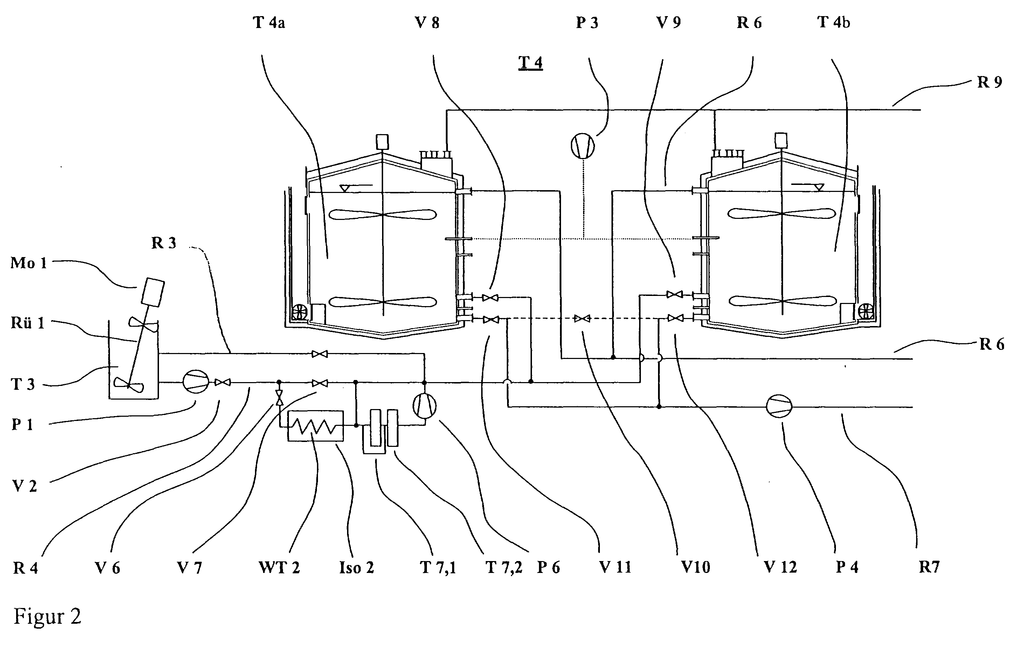

Figur 2 zeigt einen Biogasreator T4 mit einer vorgeschalteten, separaten Vormischeinrichtung in Form einer Vorgrube T3. Der Biogasreaktor T4 wird durch zwei Reaktorbehälter T 4a und T 4b gebildet, die wahlweise einzeln, parallel oder in Reihe betrieben werden können. Die Reaktorbehälter T 4a und T 4b werden im folgenden zusammen als Reaktor T4 oder als Teilreaktoren bezeichnet.

Die fertige Silage wird mit Radlader und/oder einer Greifvorrichtung aus dem Silo T1 entnommen, in einen Kipphänger oder auf ein Transportband gefüllt, zur Vorgrube T3 transportiert und in diese abgekippt. Der Transport mit dem Kipphänger erfolgt auf der Straße oder Schiene. Es kann eine Silierung in einem Silo aber auch am Ort der Biogasanlage vorhanden sein.

In der Vorgrube T3 befindet sich vorzugsweise zuvor bereits etwas Frischgülle und/oder Einstreu und/oder Festmist. Die Vorgrube T3 wird nach dem Abkippen der Silage mit Gülle und/oder Faulwasser aufgefüllt und die Mischung mit einem in der Vorgrube T3 befestigten Rührwerk Rü1, vorzugsweise ein Schneidrührwerk, homogenisiert und vorzugsweise zerkleinert, bis sich ein Trockensubstanzgehalt von vorzugsweise 10 bis 30 % einstellt.

In die Vorgrube T3 werden organische Abfälle und/oder Gülle gegeben, die vorher vorzugsweise erhitzt wurden. Hierfür dient ein Wärmetauscher WT2 der Anlage. Die im Wärmetauscher WT2 erhitzten Stoffe werden in einem Haltetank T7, der vorzugsweise mit einer Zusatzheizung versehen ist, bei vorzugsweise 70 bis 75°C für vorzugsweise 30 bis 60 min thermisch hygienisiert. Sie können über eine Pumpe P6 über eine Leitung R3 in die Vorgrube T3 und/oder von einer Leitung R4 in den Biogasreaktor T4 gepumpt werden.

Die Mischung in der Vorgrube T3 wird mit einer Dickstoffpumpe P1 über die Leitung R4 wahlweise über ein Ventil V8 oder ein Ventil V9 oder beide Ventile in einen der stehenden Teilreaktoren T 4a oder T 4b oder in beide Teilreaktoren befördert.

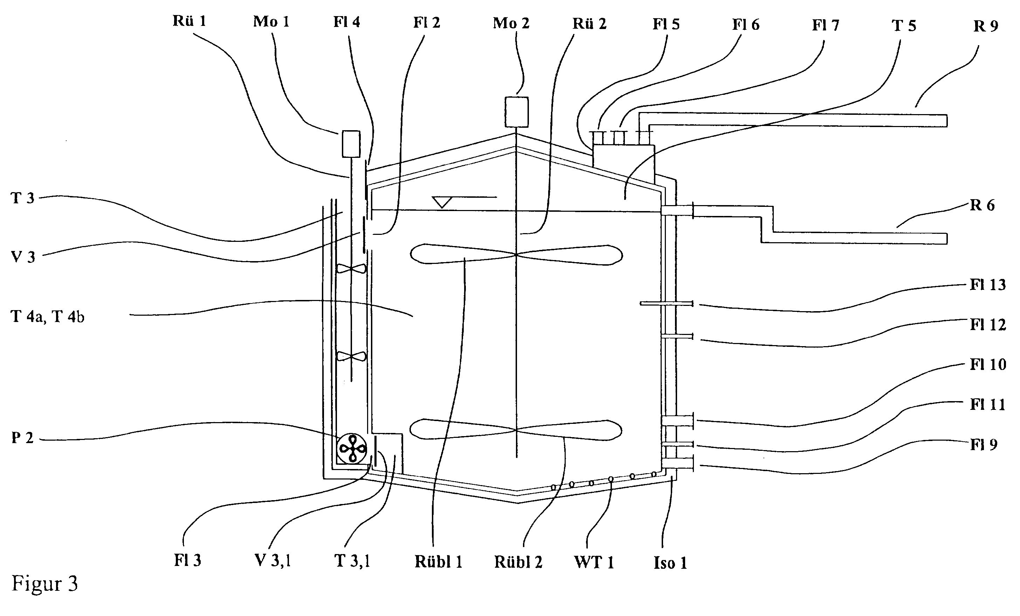

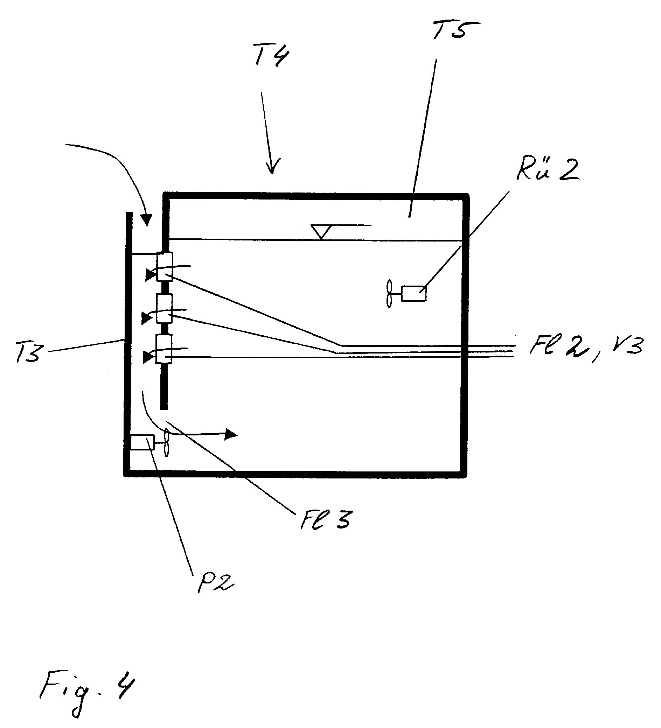

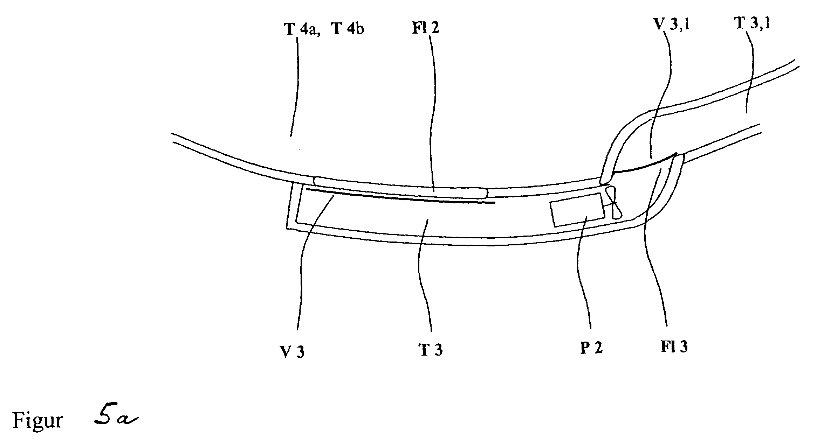

Figur 3 zeigt einen stehenden Biogasreaktor T4 mit einer integrierten Vormischeinrichtung T3. Figur 5a zeigt die Vormischeinrichtung T3 in einem zu Figur 3 senkrechten Schnitt. Die Vormischeinrichtung T3 ist unmittelbar an dem Biogasreaktor T4 als Seitenschacht ausgebildet. Der Seitenschacht ist wegen der gleichen Funktion wie die Vorgrube ebenfalls mit T3 bezeichnet. Die beiden Teilreaktoren T 4a und T 4b im Ausführungsbeispiel der Figur 2 können wie der Reaktor T4 der Figuren 3 und 4 ausgebildet, insbesondere je mit einem Seitenschacht T3 versehen sein. Eine separate Vorgrube entfällt in diesem Falle vorzugsweise. Die Pumpe P1 und Teile der Rohrleitung R4 können entfallen oder anders eingesetzt werden.

Die silierten, festen, schüttfähigen Stoffe, sowie Gülle und/oder organische Abfälle werden in den Seitenschacht T3 sehr einfach durch eine nach oben offene Einfüllöffnung eingefüllt. Bei Ausbildung des Biogasreaktors T4 in Form mehrerer Teilreaktoren mit integrierten Seitenschächten T3 werden diese Stoffe bei Parallelbetrieb in jeden der Seitenschächte T3 eingefüllt. Die leichten pflanzlichen Stoffe neigen zur Brückenbildung und lassen sich nur schwierig nach unten befördern. Die Funktion des Seitenschachtes T3 wird durch erfindungsgemäße Einrichtungen verbessert. Vorzugsweise enthält solch ein Seitenschacht T3 einen Deckel Fl 4. Der Deckel Fl 4 kann aufgeklappt als Ankippwand für feste Stoffe dienen. Der Deckel Fl 4 verschließt die Einfüllöffnung des Seitenschachts T3 vorzugsweise geruchs- und spritzwasserdicht. Im Seitenschacht T3 ist ein vorzugsweise als Schneidrührwerk ausgebildetes Rührwerk Rü1 angebracht, welches die Stoffe miteinander vermischt und vorzugsweise zerkleinert. Der stehende Behälter des Reaktors T4 weist im Seitenschacht T3 vorzugsweise zwei weitere Öffnungen Fl 2 und Fl 3 auf unterschiedlichen Höhen auf. Die zwei Öffnungen Fl 2 und Fl 3 liegen vorzugsweise diagonal übereinander, so dass eine Horizontalströmung im Seitenschacht T3 ebenfalls entsteht.

Die obere Öffnung Fl 2, die vorzugsweise 2 bis 100 cm unterhalb des Seitenschachtdeckels Fl 4 liegt und bevorzugt in mehrere über 20 bis 100% der in Behälterumfangsrichtung gesehenen Breite des Seitenschachtes T3 verteilten Teilöffnungen unterteilt ist, ist vorzugsweise verschließbar von Außen und/oder von Innen. Dazu ist als Verschluss V3 eine Klappe mit vorzugsweise Rückschlagwirkung, ein Ventil oder ein Schieber oder Schnellschlussschieber eingesetzt. Vorzugsweise ist am Boden des Seitenschachtes T3 eine Fördereinrichtung P2, beispielsweise ein förderndes Tauchmotorrührwerk oder eine Pumpe, parallel oder senkrecht zum Reaktor T4 installiert. Wenn P2 betätigt wird, strömt durch den vorzugsweise gleichzeitig geöffneten Verschluss V3 ausgefaulte Flüssigkeit aus dem Reaktor T4 in den Seitenschacht T3 auf die freie Oberfläche nach und spült die frischen Stoffe, insbesondere die festen, aufschwimmenden Pflanzenteile, direkt in P2. Die Spülwirkung kann gesteigert werden, indem der Verschluss V3 automatisch und vorteilhafterweise verzugslos öffnet, wenn in dem Reaktor T4 im Vergleich zu dem Seitenschacht T3 ein Differenzdruck aus dem Bereich von vorzugsweise 10 bis 200 cm Wassersäule erreicht ist. Faulwasser sprüht vorzugsweise in den Seitenschacht T3.

P2 befördert die Stoffe vorzugsweise so in den Reaktor T4, dass Kurzschlussströmungen zu der oder den oberen Öffnungen Fl 2 ausgeschlossen sind. Die Fördereinrichtung P2 kann so angeordnet sein, dass die Förderrichtung von P2 senkrecht oder parallel zur Behälterwand durch die Öffnung Fl 3 weist. Bei parallel zur Behälterwand des Reaktors T4 weisender Förderrichtung, wie in Fig. 5a dargestellt, ist die Ecke des Schachtes T3, in die das Rührwerk oder die Pumpe P2 fördert, vorzugsweise abgerundet, um den Reibungswiderstand zu verringern und gleichzeitig die Richtung des Stromes in den Behälter des Reaktors T4 vorzugeben. Der Radius der Abrundung ist vorzugsweise das 1 bis 8-fache des Querschnittes der unteren Öffnung Fl 3. Eine vorzugsweise in das Innere entlang der Behälterwand reichende Erweiterung T 3,1 von T3 mit dem Querschnitt von dem 0,2 bis 2-fachen von Fl 2 und einer Länge, die höchstens dem halben Umfang des Behälters des Reaktors T4 entspricht, verhindert eine Kurzschlussströmung. P2 fördert nach Beendigung des Beschickungsvorganges den Seitenschacht T3 vorzugsweise leer. Die untere Öffnung Fl 2 wird dann vorzugsweise von Außen und/oder von Innen mit einem Verschluss V 3,1 geschlossen. Die Öffnung Fl 2 kann auf gleicher Höhe wie P2 oder 2 bis 100 cm über P2 angebracht sein, was den Vorteil hätte, dass P2 nicht trocken laufen kann.

Figur 4 zeigt eine Variante einer ebenfalls mit Hilfe eines integrierten Seitenschachtes ausgebildeten Vormischeinrichtung T3. Im Unterschied zu der Vormischeinrichtung T3 der Figur 3 sind in der Seitenwand des Reaktors T4 zum Seitenschacht T3 mehrere obere Öffnungen Fl 2 auf unterschiedlichen Höhen ausgebildet. Durch die Ausbildung von mehreren, übereinander angeordneten Öffnungen Fl 2 ist eine Entleerung oder zumindest teilweise Entleerung des Reaktors T4 unter gleichzeitiger Ausnutzung des vorteilhaften Spüleffekts des Seitenschachts T3 bis zu tieferen Füllständen des Reaktors T4 möglich. Bei einer Entleerung des Reaktors T4 werden die Öffnungen Fl 2 von oben nach unten sukzessive geöffnet. Jede der mehreren auf unterschiedlichen angeordneten Öffnungen Fl 2 ist wie diejenige der ersten Ausführung ausgebildet, insbesondere in Bezug auf die Horizontale wieder wie bei der Ausführungsform der Figur 3 in mehrere Teilöffnungen unterteilt. In Bezug auf die Lage zur unteren Öffnung Fl3 gilt ebenfalls wieder das zur Ausführungsform der Figur 3 Gesagte.

Im Ausführungsbeispiel der Figur 4 ist die Fördereinrichtung P2 in dem Seitenschacht T3 so angeordnet, dass sie die Mischung aus nachfließender, ausgefaulter Flüssigkeit und neu eingefüllter Biomasse senkrecht zur Behälterwand des Reaktors T4 fördert. Eine Anordnung der Fördereinrichtung P2 mit zur Behälterwand paralleler Förderrichtung wie im Ausführungsbeispiel der Figur 3 wäre jedoch ebenfalls möglich und sogar bevorzugt. Im Unterschied zum Seitenschacht T3 der Figur 3 weist der Seitenschacht T3 der Figur 4 auch keine Erweiterung T3,1 auf. Die Verbindung zwischen dem Seitenschacht T3 und dem Reaktorinnenraum wird durch eine einfache Öffnung in Förderrichtung der Fördereinrichtung P2 in der Behälterwand gebildet. Die Ausbildung einer in das Innere des Reaktors T4 entlang der Behälterwand reichenden Erweiterung in der Art der Erweiterung T3, 1 der Figur 3 kann mit Vorteil jedoch ebenfalls vorgesehen sein. Schließlich weist der Seitenschacht T3 der Figur 4 auch keine zusätzliche Mischeinrichtung über der Fördereinrichtung P2 auf, obgleich eine Mischeinrichtung in der Art des Rührwerks Rü1 der Figur 3 ebenfalls mit Vorteil in dem Seitenschacht T3 angeordnet sein kann und in diesem Falle vorzugsweise ebenfalls die neu in den Seitenschacht T3 eingeführte Biomasse zerkleinert und den Mischeffekt mit der ausgefaulten Flüssigkeit zusätzlich verstärkt. Wie das Ausführungsbeispiel der Figur 4 zeigt, wird die Durchmischung und Spülwirkung der neuen Biomasse durch die Einleitung der ausgefaulten Flüssigkeit aus dem Reaktor T4 von oben und der im unteren Bereich des Seitenschachts T3 bevorzugt angeordneten Fördereinrichtung P2 erzielt.

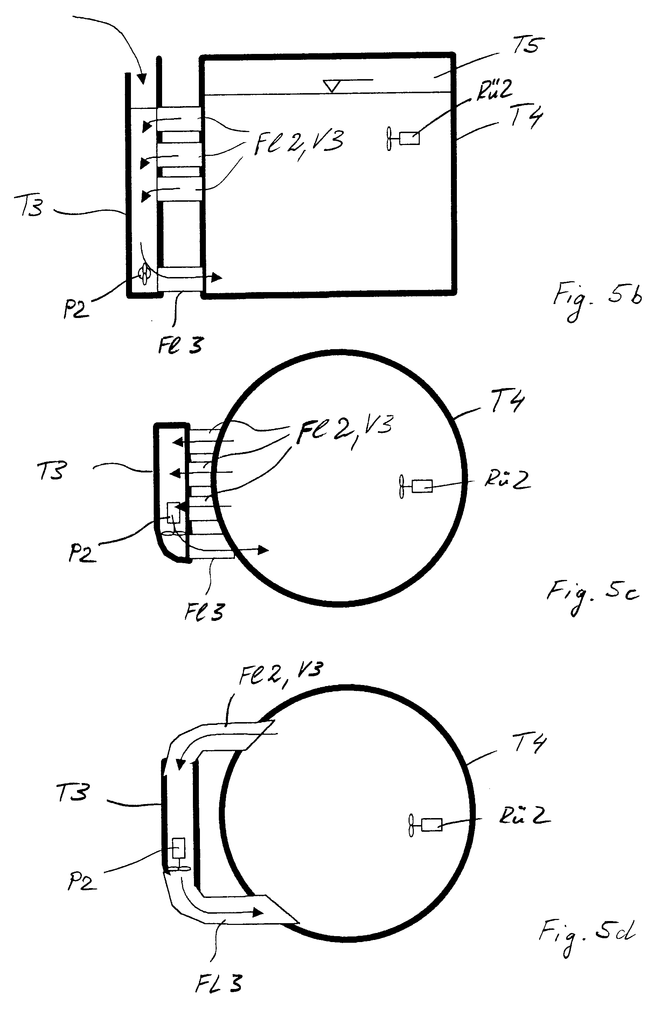

Die Figuren 5b und 5c zeigen einen Reaktor T4 mit einem stehenden Seitenschacht T3, der neben dem Reaktor T4 in einer Entfernung von höchstens einigen Metern angeordnet ist. Der Seitenschacht T3 und seine Verbindungen mit dem Reaktor T4 entsprechen dem Ausführungsbeispiel der Figur 4, wobei jedoch wegen der abgesetzten Aufstellung des Seitenschachts die Verbindungen zu dem Reaktor T4 nicht mehr durch einfache Öffnungen in der Reaktorseitenwand, sondern durch entsprechende Verbindungsleitungen Fl2 und Fl3, vorzugsweise Verbindungsrohre, gebildet werden.

Die Seitenschachtanordnung gemäß Figur 5d weist wieder nur eine Verbindungsleitung Fl2 auf, über die ausgefaulte Flüssigkeit aus dem Reaktor T4 von oben auf an der freien Flüssigkeitsoberfläche aufschwimmenden, frischen Biomasse geleitet werden kann. In Bezug auf die Anordnung von Öffnungen in der Behälterwand, die in Figur 5d in der Verbindungsleitung Fl2 zusammengefasst sind, wird wieder auf die Ausführungen zu den Figuren 3 bis 5a verwiesen. Die Verbindungsleitung Fl2 ist vorzugsweise mit einem Verschluss V3 verschließbar, für den ebenfalls wieder die Ausführungen zu den vorstehend beschriebenen Ausführungsbeispielen gelten. Die Besonderheit des Seitenschachts der Figur 5d besteht darin, dass ausgefaulte Flüssigkeit aus dem Reaktor T4 durch die obere Verbindungsleitung Fl2 hindurch im Querschnitt der Figur 5d gesehen in Richtung auf die Mündung der unteren Verbindungsleitung Fl3 in den Seitenschacht T3 einströmt. Hierfür mündet die obere Verbindungsleitung Fl2 an der Schmalseite des Seitenschachts T3, die von der unteren Öffnung für die untere Verbindungsleitung Fl3 abgewandt ist. Bereits hierdurch stellt sich im Seitenschacht T3 eine Horizontalströmung ein, welche die Fördereinrichtung P2 unterstützt.

Der Biogasreaktor T4 kann in mehrere, bevorzugt in höchstens 4 Teilreaktoren bzw. Reaktorbehälter aufgeteilt sein. Besonders bevorzugt ist er in zwei Reaktorbehälter T 4a und T 4b aufgeteilt, wobei T 4a ein stehender oder liegender zylindrischer Behälter ist. Behälter T 4b ist vorzugsweise ein stehender zylindrischer Behälter. Die Behälter des Biogasreaktors T4 stehen vorzugsweise gasseitig und hydraulisch miteinander in Verbindung. Gasseitig sind sie über eine Gasleitung R9 in Reihe geschaltet und direkt mit einem Gasspeicher T8 verbunden.

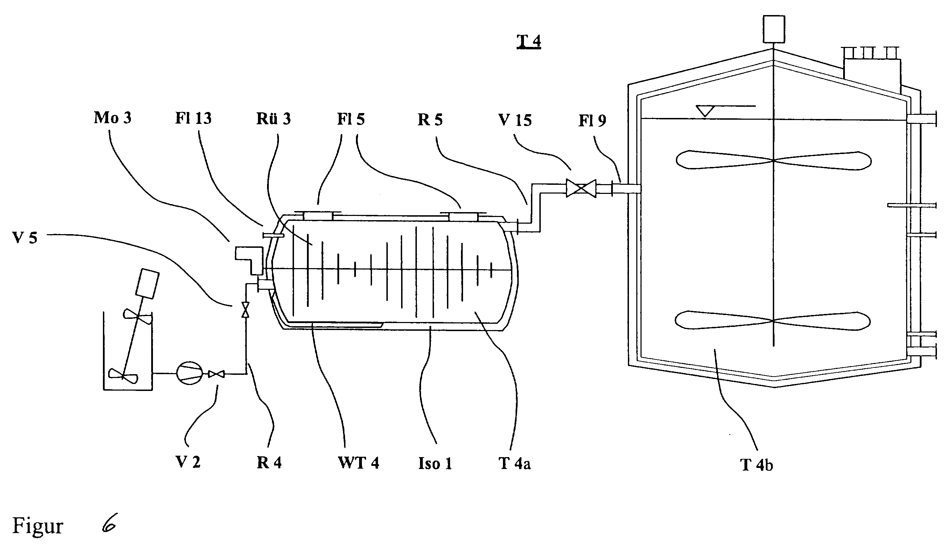

Die Figuren 2 und 6 zusammen zeigen eine komplette Biogasanlage mit Biogaserzeugung, -speicherung und -trennung.

Wenn der Reaktor, wie bevorzugt und in Fig. 2 dargestellt, aus zwei stehenden Behältern T 4a und T 4b besteht, werden die Behälter vorzugsweise durch Ansteuerung der Ventile V8, V9, V10, V11 und V12 wahlweise parallel oder in Reihe beschickt. Die Entleerung der Behälter geschieht durch Überlaufleitungen R6 direkt in einen Nachgärtank T6, der in Fig. 6 dargestellt ist, und/oder durch Abpumpen. Zum Abpumpen wird eine Pumpe P4 saugseitig über Ansteuerung der Ventile V10, V11 und V12 mit der Entleerungsleitung R7 des jeweiligen Behälters verbunden.

Mit einer strichlierten Linie ist eine Rohrleitung angedeutet, in der das Ventil V10 sitzt. Mittels dieser Verbindung und entsprechender Schaltung der Ventile V10 bis V12 können die beiden Teilreaktoren T4a und T4b wahlweise parallel oder in Reihe hintereinander betrieben werden.

Der stehende zylindrische Teilbehälter T 4a und/oder T 4b hat vorzugsweise ein Verhältnis von Höhe zu Durchmesser von 0,2 bis 4 zu 1, ist gasdicht und gasseitig vorzugsweise an der höchsten Stelle in einem Kopfraum T 5 oder in einem Mannloch Fl 5 im Kopfraum über die Rohrleitung R9 mit einem Biogasspeicher T8 für rohes Biogas verbunden (Fig. 6). Der Biogasspeicher T8 ist vorteilhafterweise in den Nachgärtank T6 integriert. Der Kopfraum T5 des Biogasreaktors T4 steht unter einem Gasüberdruck von vorzugsweise 1 bis 100 mbar und hat vorzugsweise eine Höhe von 30 bis 200 cm. Beim Abpumpen von ausgefaulter Flüssigkeit aus dem Reaktor T4 strömt Biogas aus T8 hinein und beim Zupumpen der frischen Stoffe in T8 ab, um den Druckausgleich im Kopfraum T5 des Reaktors T4 zu gewährleisten. Zudem ist der Biogasreaktor T4 mit frostsicheren, vorzugsweise hydraulisch und/oder als Berstmembran wirkenden Über- und Unterdrucksicherungen ausgestattet, die an Stutzen Fl 6 und Fl 7 vorzugsweise am Mannloch Fl 5 angebracht sind. Die Überdrucksicherung spricht vorzugsweise bei 50 bis 150 mbar, die Unterdrucksicherung bei vorzugsweise -2 bis -10 mbar zum atmosphärischen Druck an.

Der stehende zylindrische Teilbebälter T 4a und/oder T 4b enthält ein vorzugsweise langsam laufendes Rührwerk Rü 2. Die Drehzahl des Rührwerks beträgt zwischen 2 bis 100 Umdrehungen pro Minute, und die Drehrichtung ist umkehrbar. Das Rührwerk Rü 2 wird von einem vorzugsweise außerhalb des Reaktionsraums angeordneten drehrichtungsvariablen Motor Mo 2 angetrieben. Das Rührwerk Rü 3 hat vorzugsweise eine mittig angebrachte, senkrechte Welle, an der vorzugsweise zwei Rührblätter befestigt sind: Rübl 1 vorzugsweise am oberen Ende der Rührwelle unterhalb des Flüssigkeitsspiegels und Rübl 2 vorzugsweise am unteren Ende der Rührwelle in der Nähe des Bodens des Behälters. Die beiden Rührblätter bewirken eine Zerstörung von Schwimm- und Sinkschichten und homogenisieren den Inhalt. Der Rührer Rü 3 wird vorzugsweise in vorgegebenen Zeitintervallen angehalten und läuft ansonsten vorzugsweise ständig. Es handelt sich um einen vollständig durchmischten Reaktor, d.h. einen CSTR. Das Rührwerk Rü 3 kann durch ein oder zwei Tauchmotorrührwerke gebildet sein, die vorzugsweise 20 bis 200 cm unter der Oberfläche und vorzugsweise 20 bis 200 cm über dem Boden befestigt sind, was auch für die Rührblätter Rübl 1 und Rübl 2 gilt.

Im stehenden Teilbehälter T 4a oder T 4a und T 4b sind außer Öffnungen für das Rührwerk Rü 3 und die Über- und Unterdrucksicherung weitere Öffnungen für das Mannloch Fl 5 im Kopfraum T5, für ein Sichtglas an der Grenze Flüssigkeitsspiegel und Kopfraum, für Rohrleitungen zum Zu- und Abpumpen der Flüssigkeit Fl 10 und Fl 9, für Temperatur- und Druckmessungen Fl 11 und Fl 12 sowie eines Stutzens Fl 13 zur Zugabe von Chemikalien vorgesehen. Die Öffnung Fl 10 zum Zupumpen frischer Stoffe liegt vorzugsweise 50 bis 200 cm über der Reaktorunterkante. Die Öffnung Fl 9 zum Abpumpen geht von der Mitte des vorzugsweise konisch oder waagerecht gestalteten Behälterbodens oder vorzugsweise seitlich in der Behälterwand bei waagerecht gestaltetem Behälterboden ab.

Der Reaktor T4 ist rundherum thermisch mit Wärmeschutz Iso 1 isoliert, um einen k-Wert von · 5 W/m²K zu gewährleisten. In der Bodenplatte ist vorzugsweise eine Fußbodenheizung WT1 mit einer Heizleistung von 4 bis 8 Watt/(m²K) verlegt. Die Fußbodenheizung WT 1 wirkt vorzugsweise in Ergänzung zu dem Wärmetauscher WT 2 und wird vorzugsweise mit Warmwasser über eine Heizungspumpe beschickt. Mit der Fußbodenheizung werden geringe Temperaturdifferenzen zur Reaktortemperatur von vorzugsweise >5°C ausgenutzt. Der Reaktor wird bevorzugt auch dann noch beheizt, wenn beispielsweise die Zuführung von Biomasse für einige Tage unterbrochen und/oder der Wärmetauscher WT 2 nicht in Betrieb ist.

Die Beheizung des Biogasreaktors auf vorzugsweise 26 bis 36 °C geschieht vorzugsweise über den außenliegenden Gegenstrom- und/oder Kreuzstromwärmetauscher WT 2, vorzugsweise ein Rohrbündelwärmetauscher, Plattenwärmetauscher, Spiralwärmetauscher und/oder Gülle-Gülle-Wärmetauscher, der thermisch mit Iso 2 isoliert ist, um vorzugsweise einen k-Wert von · 3 W/(m²K) zu gewährleisten. Die Heizleistung des Wärmetauschers WT2 ist so dimensioniert, dass vorzugsweise der gesamte Stoffstrom der Biomassen vorzugsweise in Verbindung mit dem Wärmetauscher WT 2 und den Haltetanks / Hygienisierungsbehältern T 7,1 und T 7,2 auf vorzugsweise · 40°C oder vorzugsweise nur Teilströme wie beispielsweise Gülle und/oder organische Abfülle auf bis zu vorzugsweise 75°C erhitzt werden können und vorzugsweise mit dieser Energie der Reaktor auf die gewünschte Temperatur von vorzugsweise 26 bis 36°C gebracht werden kann. Die Energiezufuhr erfolgt vorzugsweise mit heißem Wasser und/oder Dampf von vorzugsweise · 130°C aus einer Energiestation T10 am Ort der Anlage. Besonders vorteilhaft ist es, wenn Abwärme der in der Anlage eingesetzten gekapselten Kompressoren für die Gase Biogas, Methan und Kohlendioxid für die Beheizung von T 4, T 4a, T 4b, T 7, T 7,1, T 7,2 und in WT 2, WT 3 und/oder WT 4 verwendet wird. Bekanntlich wird nur etwa ein Drittel der elektrischen Anschlussleistung in Verdichtungsarbeit, jedoch etwa 2 Drittel in Abwärme umgesetzt. Falls erforderlich, kann ein auf vorzugsweise bis 75°C erhitzter Flüssigkeitsteilstrom oder der gesamte Strom in vorzugsweise zwei bis vier, vorzugsweise wechselweise beschickten Hygienisierungsbehältern T7,1 und T7,2 bei dieser Temperatur vorzugsweise 30 bis 60 min lang gehalten werden. Die · 75°C heißen hygienisierten Stoffe werden mit der Pumpe P6 in den Gegenstromwärmetauscher WT 2, besonders bevorzugt ein Gülle-Gülle-Wärmetauscher, und/oder in den Biogasreaktor T4 und/oder die Vormischeinrichtung T3 gepumpt.

Die homogenisierte Mischung wird aus der Vormischeinrichtung T3 mittels der Dickstoffpumpe P1 auf einmal oder vorzugsweise in bis zu 3 über den Tag verteilten Chargen durch die Leitung R4, bei entsprechender Ansteuerung der Ventile V6 und V7 über den außenliegenden Wärmetauscher WT 2, in den Biogasreaktor T4 bzw. in dessen Behälter T 4a und/oder T 4b gepumpt. Bevor in T4 bzw. Behälter T 4a und/oder T 4b gepumpt wird, wird vorzugsweise die gleiche Menge aus dem Biogasreaktor abgepumpt, wenn nicht auf Überlauf geschaltet ist. Das zugepumpte Volumen wird so gewählt, dass in dem Biogasreaktor bzw. in allen Teilreaktoren von T4 ohne T6 zusammengenommen vorzugsweise eine durchschnittliche hydraulische Aufenthaltszeit von 15 bis 80 Tagen gewährleistet wird.

Figur 6 zeigt eine besonders bevorzugte Ausführungsalternative zu den stehenden Teilbehältern der Fig. 2 mit einem liegenden Teilbehälter T 4a und einem damit in Reihe geschalteten, stehenden Teilbehälter T 4b. Wenn der Reaktor T4, wie bevorzugt und in Fig. 6 dargestellt, aus einem liegenden und einem stehenden Behälter besteht, werden die Behälter hydraulisch so in Reihe geschaltet, dass der liegende Behälter T 4a über die Pumpe P1 zuerst beschickt wird. Der Überlauf fließt ohne weitere Pumparbeit in T 4b.

Der liegende zylindrische Behälter T 4a mit kreisförmigem Querschnitt ist auf Stützen aufgestellt, die vorzugsweise 2 - 4 m auseinander liegen. Er hat ein Volumen von 100 bis 200 m³, einen Durchmesser von 3 bis 4m und ist aus 3-4mm Stahlblech gefertigt. Er enthält ein durchgehendes horizontales Rührwerk Rü 3, dessen Rührflügel tragende Welle an den beiden Enden und zusätzlich in Abständen von vorzugsweise 2 - 4 m gelagert ist. Die Reaktorenden sind vorzugsweise als Böden in Klöpperform gestaltet. Der Behälter T 4a enthält an jedem Ende ein Mannloch Fl 5. Vorzugsweise das erste Drittel des Behälters ist auf der Unterseite mit einem Heizmantel WT 4 umschlossen. Der Stutzen Fl 13 dient zur Zugabe von Chemikalien und ragt vorzugsweise 10 bis 20 cm in den Behälter hinein. Auf der Rührerwelle sind in Abständen von vorzugsweise 0,5 bis 2 m Rührarme befestigt. Jeder Rührarm ist gegenüber seinem benachbarten Rührarm um einen Winkel von 18° bis 36° versetzt auf der Welle angeordnet. Die gemischten Stoffe mit vorzugsweise 5 bis 40 % Trockensubstanz werden mit der Pumpe P1 über die Rohrleitung R4 und Ventil V5, das vorzugsweise eine Rückschlagklappe ist, in den liegenden Fermenter T 4a gepumpt, durchlaufen den Fermenter in vorzugsweise 4 bis 10 Tagen und treten vorzugsweise zusammen mit dem gebildeten Gas durch ein Rohr R5 und eine Rückschlagklappe V 15 in den stehenden Fermenter T 4b ein. Durch diese Vorgehensweise ist der liegende Fermenter T 4a ständig gefüllt, steht unter dem Druck, der durch die Flüssigkeitssäule im senkrechten Reaktor T 4b vorgegeben ist, und benötigt keinen Gasdom. Ansammlungen von abgesetzten Stoffen werden vorzugsweise über eine am Ende angebrachte nicht dargestellte Schnecke gelegentlich abgezogen. Die Förderung durch den Behälter T 4a erfolgt vorzugsweise durch die Pumpe P1 und nicht durch das Rührwerk Rü 3.

Das Rührwerk Rü 3 ist vorzugsweise so ausgebildet, dass es nur eine radiale Rührwirkung entfaltet und dem Behälterinhalt keinen Vorschub verleiht und dass eine fördernde Wirkung in dem Behälter T 4a vorzugsweise nur durch eine Stoffzuführung ausgeübt wird. Der liegende Behälter T 4a ist auf der Unterseite mit einem Heizmantel WT 4 umschlossen, der 20 bis 80 % der Unterseite des liegenden Behälters T4,1 bedeckt. Der liegende Behälter T 4a enthält keinen Gasdom und entleert das gebildete Gas zusammen mit Flüssigkeit über eine Rückschlagklappe V 15 in den zweiten Behälter T 4b, wobei eine Austrittsöffnung an einem oberen Ende des liegenden Behälters T 4a angeordnet ist.

Sowohl der liegende Reaktor T 4a als auch der stehende Reaktor T 4b können mit einem integrierten oder abgesetzten Seitenschacht der beanspruchten Art und den anhand von Ausführungsbeispielen bevorzugten weiteren Merkmalen ausgestattet sein.

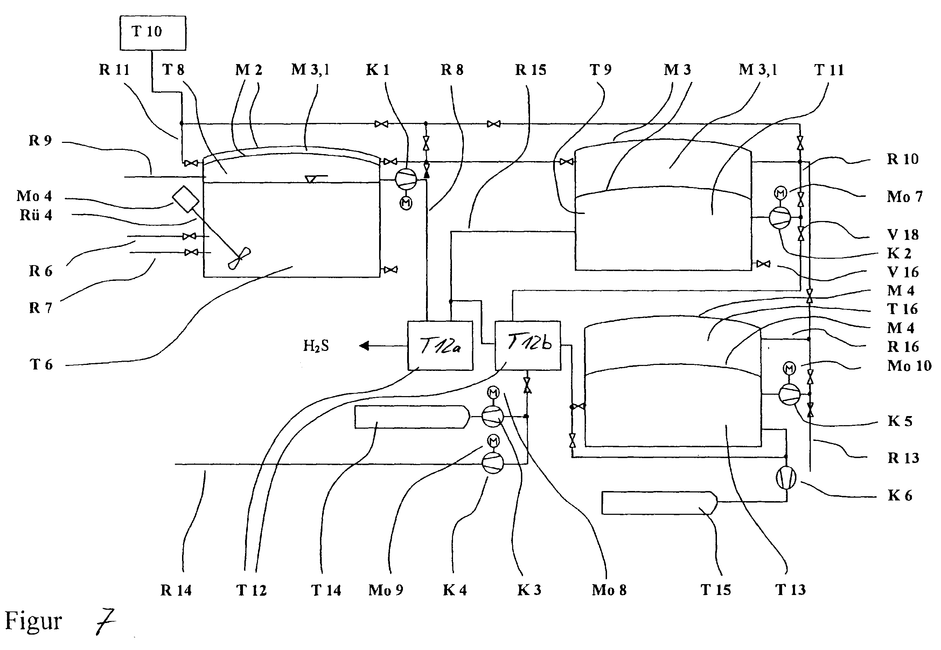

Figur 7 zeigt die Speicherung und Trennung des Biogases.

Der Biogasspeicher T8 für rohes Biogas ist vorzugsweise ein Niederdruckgasspeicher, der separat in einer vorzugsweise feuerhemmenden Umhüllung untergebracht ist. T8 ist in den Nachgärtank T6 als Abdeckung integriert. Der Nachgärtank T6 ist mit einem von einem Motor Mo 4 angetriebenen, seitlich angebrachten Rührwerk Rü 4 ausgerüstet. Der Speicher T8 wird von einer Membran M2, insbesondere einer Folie luftundurchlässig abgeschlossen. Der Nachgärtank T6 bildet ein Teil des Reaktors T4.

In den bisherigen Biogasspeichern ist ein Gasraum über einer abschließenden Membran der Luft ausgesetzt, absichtlich belüftet und/oder unter Luftdruck gesetzt, um dem Biogas unter der Membran einen Druck zu verleihen. Zur Herstellung von vermarktungsfähigem, erdgasgleichen Biomethan und Kohlendioxid ist erfindungsgemäß hingegen der Kontakt zu Luft und insbesondere Sauerstoff verhindert. An die Reinheit von Kohlendioxid werden besonders hohe Anforderungen gestellt. Deshalb wird für die Membran M2 eine sehr geringe Permeabilität für Luft bei 0 bis 30°C von vorzugsweise höchstens 150 cm³/(m²bar24h) gefordert. Bei preisgünstigen Membranen mit Permeabilitäten im oberen, erfindungsgemäß noch zulässigen Bereich wird vorzugsweise ein Schutzraum M 3,1 ausgebildet, um die Isolation zu verbessern. Dieser Schutzraum M 3,1 wird erfindungsgemäß statt mit Luft vorzugsweise mit Prozessgasen, vorzugsweise mit Biogas und/oder Kohlendioxid, gespült. Der Schutzraum M 3,1 kann mehrfach unterteilt sein und/oder insbesondere durch mehrere Schutzräume M 3,1 übereinander gebildet werden. Vorgefertigte Schutzräume können an Gasspeichern, die vorzugsweise als Säcke und/oder Kissen ausgebildet sind, angebracht werden. Biogas dient nach dem Durchgang durch den Schutzraum M 3,1 vorzugsweise zur Prozessenergieerzeugung in einer in der Anlage integrierten Energiestation. Durchgeleitetes Kohlendioxid dient vorzugsweise als Dünger in Treibhäusern und/oder zur Entwesung von Lagern, wo der Sauerstoffgehalt nicht stört.

Ein insgesamt sehr geringer Durchgang von Luft wird vorzugsweise gewährleistet durch eine Edelstahlmembran, eine metallbedampfte Kunststofffolie und/oder eine wenigstens zweischichtige Membran M2. Zwei Schichten werden vorzugsweise gebildet durch flexible Kunststofffolien oder durch eine gasdichte starre Behälterabdeckung mit darunter liegender flexibler Folie, wobei der Schutzraum M 3,1 vorzugsweise mit rohem Biogas, entschwefeltem Biogas oder Kohlendioxid gefüllt und vorzugsweise durchströmt und das Biogas nach Verlassen des Schutzraumes M 3,1 vorzugsweise in der Energiestation verbraucht wird. Der Schutzraum M 3,1 hat ein konstantes oder variables Volumen. Ein konstantes Volumen des Schutzraumes M 3,1 wird vorzugsweise bei Gassäcken und Gaskissen erreicht, indem die beiden vorzugsweise flexiblen und/oder dehnbaren Folien durch Abstandshalter getrennt sind. Durch die Form der Abstandshalter kann ein kontrollierter Gasfluss unterstützt werden, um Kurzschlussströmungen zu unterbinden. Die Abstandshalter können gasdicht oder porös mit einem Porenvolumen von bis zu 99,9 % sein. Der Schutzraum M 3,1 kann in mehrere Räume unterteilt sein, die vorzugsweise je einen eigenen Gaszutritt und Gasausgang haben. Der Gasspeicher T8 kann, wie die weiteren Gasspeicher der Anlage ebenfalls, mehrere übereinander angebrachte Lagen von Schutzräumen enthalten, um die Isolation gegen Lufteintritt zu verbessern.

Ein Schutzraum M 3,1 mit einem variablen Schutzraumvolumen stellt sich bei der Kombination Membran/starre Platte ein, beispielsweise zwischen einer in einem starren Behälter befestigten flexiblen Membran und den Seitenwänden und dem Dach des Behälters, weil sich die flexible und/oder dehnbare Membran, insbesondere Folie, dem Füllungsgrad anpasst. Dies führt dazu, dass der Schutzraum größer wird, wenn das Gasspeichervolumen kleiner wird und umgekehrt. Die Gesamtgasmenge an Gas im Schutzraum M 3,1 und im Speicher T8 bleibt gleich.

Durch die Erfindung wird vorzugsweise Luftzutritt in das gespeicherte rohe und/oder entschwefelte Biogas verhindert. Außerdem wird ein Überdruck von vorzugsweise 1 bis 100 mbar im Schutzraum M 3,1 und im gespeicherten Biogas erzeugt, der bei Gasspeichern mit variablem Schutzraumvolumen die Arbeit eines Gebläses K 1 und/oder K 2 unterstützt.

Zusätzlich zu dem als Rohgasspeicher dienenden Speicher T8 weist die Biogasspeicherung einen separaten Reingasspeicher T11 auf. In dem Reingasspeicher T11 wird entschwefeltes Biogas gespeichert. Über dem Reingasspeicher T11 wird mittels einer für die Belange der Praxis für Sauerstoff nicht permeablen Membran M3 wieder ein Schutzvolumen M 3,1 gebildet, das mit dem Schutzvolumen M 3,1 des Rohgasspeichers T8 ständig verbunden oder bei Bedarf verbindbar ist.

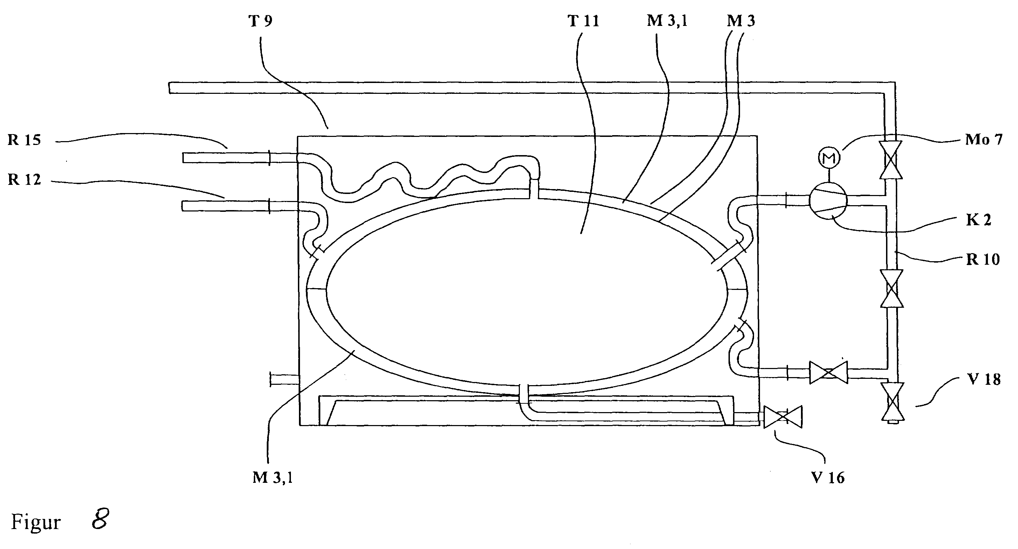

In Bezug auf den Reingasspeicher T11 und dessen Zwischenvolumen M 3,1 wird ergänzend stets auch auf Fig. 8 verwiesen. Der Reingasspeicher T11 ist als doppelwandiges Gasspeicherkissen oder -sack mit innerem Speicher T11, umgebendem flexiblen Zwischenraum M 3,1 und diesen umgebende, feuerfeste Umhüllung T9 ausgebildet. In Bezug auf die doppelschichtige Membran M3 und das von der Membran M3 gebildete Schutzvolumen M 3,1 gilt das zur Membran M2 und deren Schutzvolumen M 3,1 Gesagte gleichermaßen. Sämtliche Gasspeicher der Anlage können wie der Reingasspeicher T11 ausgebildet sein. Die Membran M3 ist auf vorzugsweise 40 bis 60% der Höhe der bevorzugt zylindrischen, senkrecht stehenden Umhüllung T9 befestigt.

Vorzugsweise ein Teilstrom des durch einen Motor angetriebenen Rohgasgebläses K 1 und/oder des durch einen Motor angetriebenen Reingasgebläses K 2 und/oder des durch einen Motor Mo 10 angetriebenen CO₂-Gebläses K 5 geförderten Gases wird in wenigstens einen der Zwischenräume M 3,1 zwischen M 2 und/oder M 3 in den Gasspeichern T8 und/oder T11 in Reihe oder parallel geleitet. Der Gasspeicher T8 ist vorzugsweise mit einer Fackel verbunden, welche plötzliche Überschüsse an Biogas abfackeln kann. Wäre der Gasspeicher T8 separat aufgestellt, so wäre vorzugsweise am tiefsten Punkt ein Ventil angebracht, wie Ventil 16 des Speichers 11, über das Kondensat abgezogen wird. Die flexible Hülle M3 des Gasspeichers T11 ist vorzugsweise mit einer Vorrichtung verbunden, die den Füllstand anzeigt. Das saugseitig mit dem Gasraum des Biogasspeichers T8 verbundene Gebläse K 1 sorgt für den notwendigen Vordruck im Rohgas für eine bevorzugte Weiterleitung zumindest eines Teilstromes in den Zwischenraum M 3,1 von M 3 und/oder zur Energiestation und/oder zu einer Gastrennungseinrichtung T 12.

Vorteilhaft ist es, wenn Schwefel bei einer Teilentschwefelung als Sulfid ausgefällt wird, was vorzugsweise durch Zugaben von Eisen-III als Hydroxid oder Chlorid bewirkt wird. Vorzugsweise wird die H₂S-Konzentration im rohen Biogas auf 1 bis 500 ppm gesenkt, indem über eine Dosierpumpe P3 Eisen-III in T 4a und/oder T 4b, vorzugsweise proportional zum Schwefelgehalt des zugeführten frischen Stoffgemisches und/oder des Biogasvolumenstroms, zugegeben wird. Die Zugabe kann besonders vorteilhaft direkt in die Vormischeinrichtung T3 erfolgen.

Vorteilhafterweise bildet diese Entschwefelung eine Vorstufe, und die vollständige Entschwefelung des Biogases erfolgt in einer ersten Trennstufe der Gastrennungseinrichtung T 12. Das so entschwefelte Biogas (Reingas) wird vorzugsweise in den separaten Gasspeicher T 11 gepumpt und steht von dort aus der Gastrennungseinrichtung T 12 und/oder vorzugsweise nach Durchgang durch die Zwischenräume M 3,1 der Membranen M2 und/oder M3 der Energiestation zur Verfügung.

Die Energiestation T10 entnimmt erfindungsgemäß rohes und/oder entschwefeltes Biogas über das Gebläse K1 aus dem Biogasspeicher T8 für rohes Biogas, über Gasleitung R11 aus dem Zwischenraum M 3,1 von T8, aus dem Zwischenraum M 3,1 des Gasspeichers T 11 für entschwefeltes Biogas und/oder direkt aus T 11 über das Gebläse K 2 und Gasleitung R 10.

Die Energiestation T10 besteht vorzugsweise aus einem Blockheizkraftwerk (BHKW) des Typs Ottomotor oder Zündstrahler, das wärmeseitig mit einer Temperaturspreizung von vorzugsweise 70 bis 130°C arbeitet, einer Brennstoffzelle und/oder einer Absorptionskältepumpe und/oder einem Heizkessel. Das in der Energiestation untergebrachte BHKW und/oder die Brennstoffzelle hat die Aufgabe, so viel Energie an Strom, Wärme und/oder Kälte aus Biogas zu erzeugen, dass vorzugsweise der Energiebedarf der Biogasanlage und der Gastrennungseinrichtung an Strom, Kälte und/oder Wärme gedeckt wird. Wenn es wirtschaftlich ist, können zusätzlich auch landwirtschaftliche Betriebe mit Strom, Wärme und/oder Kälte beliefert werden. Zusätzlich oder alternativ zu dem BHKW ist vorzugsweise eine Absorptionswärme-/kältepumpe und/oder ein Heizkessel aufgestellt, der vorzugsweise die benötigte Wärme und/oder Kälte aus Biogas erzeugt. Es ist auch schon vorteilhaft, wenn die Energiestation nur den Bedarf an Prozesswärme und Prozesskälte deckt und der Prozesselektrizitätsbedarf aus dem öffentlichen Netz gedeckt wird. Bei vorzugsweise gleichzeitiger Nutzung von Wärme, beispielsweise zur Erwärmung von Stoffen in den Wärmetauschern, und Kälte, beispielsweise bei der Kondensation von Feuchte und bei der CO2-Kompression aus der Absorptionswärme-/kältepumpe, ist der Wirkungsgrad um den Faktor 1,1 bis 1,9 besser als der eines Brennwertkessels. Strom wird vorzugsweise aus dem Netz bezogen, wenn keine Kraft-Wärme-Kopplung installiert ist. Die Energiestation ist in einem Container untergebracht, läuft vorzugsweise vollautomatisch über eine eigene EMSR- und SPS-Anlage und wird über den Füllstand der Biogasspeicher, den Gasvolumenstrom, den Methangehalt und/oder den Energiebedarf der Biogasanlage an vorzugsweise Strom, Wärme und/oder Kälte kontrolliert. Das BHKW ist vorzugsweise wärmegeführt, um die Temperatur im Biogasreaktor, im außenliegenden Wärmetauscher, bei der Hygienisierung und/oder in der Gastrennungseinrichtung aufrechtzuerhalten und die Energie bereitzustellen.

Die Biogastrennungseinrichtung T 12 entnimmt Biogas vorzugsweise aus dem Gasspeicher T8 für rohes Biogas über das Gebläse K 1. In einer Entschwefelungsstufe T12a, die einer Kältefalle zur Kondensatentfernung folgt, wird H2S entfernt. Die Entschwefelung in der Gastrennungseinrichtung T 12 wird vorzugsweise zusätzlich zu einer Entschwefelung mittels Chemikalienzugabe im Biogasreaktor T4 durchgeführt, beispielsweise der beschriebenen Eisen-III Zugabe. Die Entschwefelung erfolgt in diesem Fall in zwei Stufen. In der Gastrennungseinrichtung T 12 wird der Schwefelgehalt des Biogases auf 5 ppm oder weniger vermindert. Im Reaktor T4 erfolgt eine Entschwefelung auf vorzugsweise 5 bis 500 ppm. Die Entschwefelung in der Gastrennungseinrichtung kann nach einer anderen bevorzugten Ausführung auch die einzige Art der Entschwefelung sein. Auch in diesem Falle wird der Schwefelgehalt im entschwefelten Biogas auf 5 ppm oder weniger gesenkt. Vorzugsweise wird das gesamte Biogas entschwefelt und nicht nur der Teil, der nicht in der Energiestation genutzt wird. Das in der der Entschwefelungsstufe entschwefelte Biogas kann in dem separaten Gasspeicher T 11 für entschwefeltes Biogas zwischengespeichert werden. Das entschwefelte Biogas wird direkt nach der Entschwefelungsstufe wahlweise entweder einer nachfolgenden CH4-CO2-Trennungsstufe T12b oder dem Reingasspeicher T 11 zur Zwischenspeicherung zugeführt oder es werden zwei Teilströme gebildet, einer zur Trennstufe T12b und einer zu T11. Aus T 11 wird es über das Reingasgebläse K 2 und die Gasleitung R 10 bei geöffnetem Ventil V 18 für die CH4-CO2-Trennung entnommen.

Die Gastrennungseinrichtung T 12 ist vorzugsweise in sich vollständig automatisch geregelt und gesteuert, vorzugsweise durch eine EMSR- und SPS-Anlage. Die Gastrennungseinrichtung T 12 besteht vorzugsweise aus bei wechselndem Druck arbeitenden PSA-Modulen, insbesondere Molekularsieben und/oder absorbierenden Flüssigkeiten, zur Anreicherung von Methan bzw. Abtrennung von Kohlendioxid. Den PSA-Modulen vorgeschaltet sind vorzugsweise jodidbeaufschlagte Aktivkohlefilter zur Adsorption von Schwefelwasserstoff und Geruchsstoffen und Molekularsiebe für halogenierte Bestandteile. An die Stelle der PSA-Module können bei konstantem und niedrigem Überdruck arbeitende Membranmodule treten, die aus dem Biogas selektiv Schwefelwasserstoff und/oder Kohlendioxid entfernen. Sie bestehen vorzugsweise aus hydrophoben, mit Flüssigkeit durchströmten Membranen, die mit dem Biogas vorzugsweise im Kreuz- oder Gegenstrom angeströmt werden. Die Gase diffundieren durch die Membran in die Flüssigkeit, wobei vorzugsweise in der ersten Trennungsstufe Schwefelwasserstoff und in der zweiten Trennungsstufe Kohlendioxid von der für diesen Zweck jeweils ausgewählten Flüssigkeit vorzugsweise selektiv absorbiert werden.

Die aus der Gastrennungseinrichtung T 12 austretenden Gase Biomethan und Kohlendioxid haben eine Reinheit von vorzugsweise mindestens 95,0 Vol%, besonders bevorzugt mindestens je 99%. Der Methanverlust beträgt vorzugsweise weniger als 5%. Erdgasgleiches Biomethan wird vorzugsweise mit Tetrahydrothiophen (THT) in einer Konzentration von vorzugsweise über 10 mg/m³ in einer nicht dargestellten Odierstation odoriert und entweder über einen mit dem Motor Mo 8 angetriebenen, vorzugsweise zwei- bis dreistufigen Kompressor K3 auf vorzugsweise 250 bar komprimiert und in Druckflaschen T 14 abgefüllt und/oder über einen mit dem Motor Mo 9 angetriebenen Kompressor K4 auf einen Druck über Atmosphärendruck, vorzugsweise auf einen Druck im Bereich von 100 mbar bis 100 bar, komprimiert und über eine Volumenstrommessung am Ort der Anlage durch eine Rohrleitung R 14 in ein Erdgasnetz eingespeist. Die Leitgröße für die Einspeisung in das Erdgasnetz und damit für die Ansteuerung der Gastrennungseinrichtung ist vorzugsweise der CH₄-Gehalt und/oder die Methanzahl und/oder der Wobbeindex des einzuspeisenden Biomethans. Der Wobbeindex liegt vorzugsweise zwischen 10 und 15 kWh/m³. Wird der vorgegebene Wert für die Leitgröße nicht erreicht, so wird das Biomethan in die Gastrennungseinrichtung T 12 zurückgeführt oder vorzugsweise in den Gasspeicher T8.

Kohlendioxid wird vorzugsweise über einen für Luft nicht permeablen Niederdruckspeicher T 13 als Zwischenpuffer geleitet. Aus dem Speicher T 13 wird das Kohlendioxid mit einem motorangetriebenen Kompressor K6 als Flüssig-CO₂ in Druckbehälter T 15, beispielsweise Druckflaschen oder Tankwagen, abgefüllt und/oder in Rohrleitungen für flüssiges CO₂ eingeleitet und/oder als Gas aus dem Gasspeicher T 13 über das Gebläse K5 in Rohrleitungen zum Transport für gasförmiges CO₂ abgegeben. Das Gebläse K5 leitet vorzugsweise über ein Ventil V 19 einen Teilstrom in den Zwischenraum M 3,1 der vorzugsweise doppelschichtigen Membran M 3, um in dem Zwischenraum M 3,1 einen stabilen Überdruck von vorzugsweise 1 bis 100 mbar zu erzeugen. Überschüssiges CO₂ wird in die Umgebung abgelassen. Eine Membran M4 ist mehrwandig ausgeführt, im Ausführungsbeispiel doppelwandig, und bildet einen mit CO₂ gefüllten Zwischenraum. Für die Membran M4 gilt das zu dem Membranen M2 und M3 Gesagte.

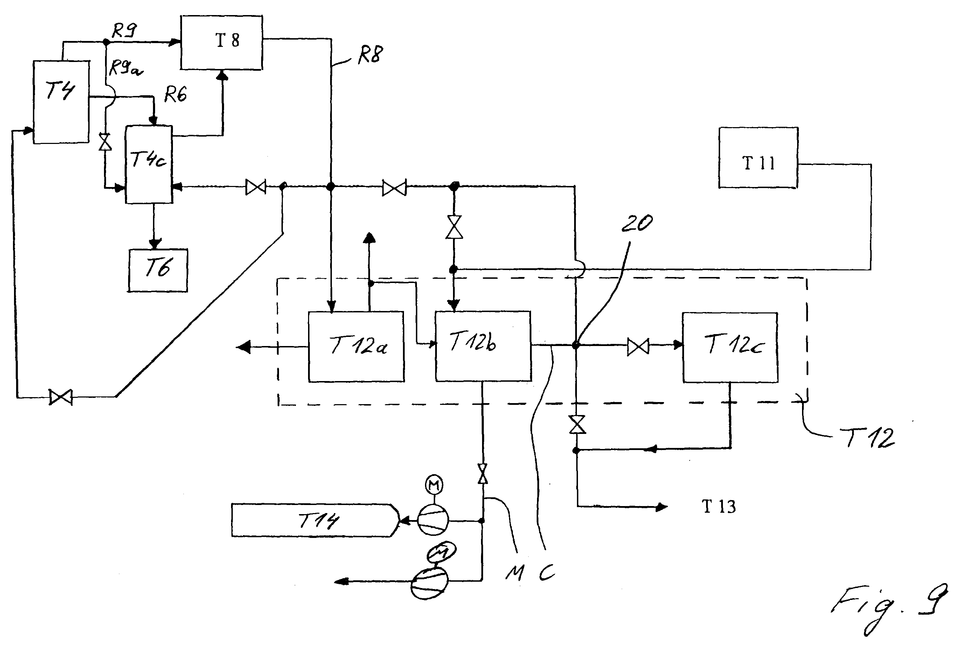

Figur 9 zeigt eine Gastrennungseinrichtung T12 und deren Zu- und Ableitungen. Die Gastrennungseinrichtung T12 der Figur 9 ist gegenüber der Gastrennungseinrichtung T12 der Figur 7 um eine zweite Methan-CO₂-Trennstufe T12c erweitert. Die Gastrennungseinrichtung T12 der Figur 9 kann in sämtlichen Ausführungen der Erfindung alternativ zu der vereinfachten Ausführung der Figur 7 verwendet werden. Das vorstehend zur Gastrennungseinrichtung T12 Gesagte gilt daher gleichermaßen auch für die Gastrennungseinrichtung T12 der Figur 9. Insbesondere ist die Einbettung in die Gesamtanlage mit Ausnahme der nachstehend beschriebenen Besonderheiten die gleiche wie in Figur 7.

Insgesamt weist die Gastrennungseinrichtung T12 der Figur 9 drei hintereinander geschaltete Trennstufen T12a, T12b und T12c auf. T12a bildet die Entschwefelungsstufe und die beiden Trennstufen T12b und T12c sind Methan-CO₂-Trennstufen, für die je die vorstehenden Ausführungen zu solchen Trennstufen T12a und T12b gelten. Jede der beiden Trennstufen T12b und T12c wird durch mehrere PSA-Kolonnen gebildet, die je mit Molekularsieben oder Flüssigkeit gefüllt sind, die selektiv Kohlendioxid absorbieren und Methan hindurchströmen lassen. Die jeweils mehreren PSA-Kolonnen einer der Trennstufe T12b und T12c werden batchweise betrieben, wobei durch Parallelschaltung und zeitlich gestaffelte Beschickung der mehreren Kolonnen in der jeweiligen Trennstufe eine Vergleichmäßigung im Produktstrom erzielt wird.

In den PSA-Kolonnen der ersten Methan-CO₂-Trennstufe T12b werden der methanreiche Teilstrom M und ein kohlendioxidreicher Teilstrom C in an sich bekannter Weise in einem PSA Verfahren erhalten. Der methanreiche Teilstrom M wird, wie bereits beschrieben, in T14 gespeichert, oder unmittelbar in ein festverlegtes Methanrohrleitungsnetz eingespeist.

In den PSA-Kolonnen der ersten Methan-CO₂-Trennstufe T12b wird der kohlendioxidreiche Teilstrom C bei der Desorption und einer anschließenden Evakuierung gebildet. Bei dem PSA-Verfahren wird Kohlendioxid bei einem Druck von 6 bis 8 bar absorbiert und bei einer anschließenden Drucksenkung desorbiert. Zum Ende der Desorption wird ein geringer Unterdruck angelegt, es wird somit evakuiert. Zu Anfang der Desorption enthält der kohlendioxidreiche Teilstrom C das meiste Methan in einer relativ hohen Konzentration von 5 bis 10 Vol%. Ferner enthält der Teilstrom C auch noch andere im Biogas enthaltene Begleitstoffe, die in einem Vorfilter vor der ersten Methan-CO2-Trennstufe T12b oder in der Trennstufe T12b selbst zurückgehalten worden sind und bei der Desorption ebenfalls desorbiert werden. Die Konzentration dieser Begleitstoffe, beispielsweise flüchtige Fettsäuren, Aldehyde, Ketone, Silane, Alkohole usw., hängt eng mit der Zusammensetzung der Inputstoffe und den Ausfaulungsbedingungen im Bioreaktor T4 zusammen. Schon die Ausfaulung von beispielsweise Apfelsinenschalen, Friteusefetten, tierischen Fetten, Kosmetika usw. kann zu flüchtigen höher- und niedermolekularen, leicht bis schwer kondensierbaren Spurenstoffen im Biogas führen. Die zusätzliche oder alleinige Ausfaulung solcher Stoffe, insbesondere in Kombination mit einer hohen Raumbelastung von über 5kg organischer Trockenmasse pro m³ Reaktorvolumen und Tag, ergibt mehr Begleitstoffe im Biogas als die Ausfaulung reiner Gülle und reiner Energiepflanzen. Mit fortschreitender Desorption nimmt der Gehalt an Methan im kohlendioxidreichen Teilstrom C und auch der Gehalt von Begleitstoffen ab, d.h. es nimmt die Reinheit des Kohlendioxids zu.

In einer die zwei Trennstufen T12b und T12c verbindenden Leitung ist eine Verzweigung 20 vorgesehen, durch die hindurch der kohlendioxidreiche Teilstrom C wahlweise der nachgeschalteten Trennstufe T12c, unmittelbar dem Kohlendioxidspeicher T13 oder in eine frühere Verfahrensstufe zurückgeführt werden kann. Die in den jeweiligen Verbindungsleitungen eingezeichneten Ventile werden entsprechend geschaltet. In der Verbindungsleitung zwischen den Trennstufen T12b und T12c ist vor der Verzweigung 20 eine Messeinrichtung angeordnet, mit der der Restgehalt an Methan im kohlendioxidreichen Teilstrom C ermittelt wird.

Zu Beginn und im anfänglichen Verlauf der Desorption ist der Restgehalt an Methan im Teilstrom C meist so groß, dass der kohlendioxidreiche Teilstrom C in eine oder mehrere der früheren Verfahrensstufen zurückgeführt wird. Dementsprechend sind die beiden Ventile in den Leitungen zur Trennstufe T12c und zum Kohlendioxidspeicher T13 geschlossen. Im Verlaufe der Desorptions- und Evakuierungsphase sinkt der Restgehalt an Methan im Teilstrom C. Wird ein vorgegebener Restgehalt unterschritten, vorzugsweise 1 Vol.% Restgehalt an Methan, so wird durch entsprechende Schaltung der Ventile der kohlendioxidreiche Teilstrom C der zweiten Methan-CO2-Trennstufe T12c zugeführt. In dieser Trennstufe T12c wird das im Teilstrom C enthaltene Kohlendioxid aufkonzentriert. Der Strom mit dem aufkonzentrierten Kohlendioxid wird anschließend aus T12c zu dem Kohlendioxidspeicher T13 geleitet. Sollte die Kohlendioxidreinheit im Teilstrom C bereits so hoch sein, dass der Teilstrom C als Kohlendioxidgas oder, nach Verflüssigung, als Flüssig-CO2 verkauft werden kann, so wird durch entsprechende Schaltung der Ventile der Teilstrom C unmittelbar zu dem Kohlendioxidspeicher T13 geleitet.

Ist der kohlendioxidreiche Teilstrom C aus der zweiten Methan-CO2-Trennstufe T12b mit Methan und/oder anderen Spurenstoffen noch hoch beladen, wie insbesondere zu Beginn der Desorption, so werden die Ventile in den Leitungen zur Trennstufe T12c und zum Kohlendioxidspeicher T13 geschlossen, und es erfolgt eine Rückführung in eine frühere Verfahrensstufe. Durch solch eine Rückführung kann zusätzlich auch der Methanverlust stark reduziert werden. Ferner können eventuelle organische Begleitstoffe durch beispielsweise anaeroben Abbau oder physikalisch chemische Reaktionen abgetrennt werden, um deren Anreicherung zu verhindern. Ob die Konzentration eines Begleitstoffes ermittelt werden kann, hängt natürlich von der Ausbildung der Messeinrichtung vor der Verzweigung 20 ab. Das Vorhandensein und die Konzentration organischer Begleitstoffe kann alternativ auch aufgrund von Erfahrungswerten abgeschätzt werden. Grundsätzlich gilt dies auch für den Restgehalt an Methan. Eine Messung einer Restkonzentration ist daher nicht unumgänglich erforderlich, sondern lediglich vorteilhaft. Die Rückführung erfolgt insbesondere bei hohen Methanrestgehalten, vorzugsweise bei Methanrestgehalten von wenigstens 1 Vol. %, da in solch einem Fall auch noch die Methangewinnung lohnt. Die Rückführung erfolgt aber auch, falls ein Begleitstoff mit solch einem hohen Gehalt im Teilstrom C enthalten ist, dass eine ausreichende Anreicherung von Kohlendioxid in der nachgeschalteten Trennstufe T12c nicht möglich oder nicht wünschenswert ist.

Wird beispielsweise lediglich ein hoher Methanrestgehalt festgestellt, so wird der Teilstrom C vorzugsweise in die erste Methan-CO₂-Trennstufe T12b zurückgeführt. Wird ein vorgegebener Gehalt eines anderen organischen Begleitstoffs überschritten, so erfolgt stattdessen vorzugsweise die Rückführung in ein Organikfilter zum Herausfiltern solcher Begleitstoffe. Ist das Organikfilter vor der Entschwefelungsstufe T12a angeordnet, wie dies üblicherweise der Fall ist, so erfolgt die Rückführung durch Zumischung zu dem Gasstrom in der Leitung R8 aus dem Gasspeicher T8 für rohes Biogas. Insbesondere für den Fall, dass der Gehalt eines weiteren Begleitstoffs oder mehrerer weiterer Begleitstoffe einen vorgegebenen Wert überschreitet, erfolgt ebenso bevorzugt stattdessen oder als Teilstrom eine Rückführung in einen oder mehrere Behälter des Bioreaktors T4 oder in einen oder mehrere Festbettreaktoren T4c. Letztere sind vorzugsweise als senkrecht stehende Festbettreaktoren, insbesondere als Füllkörperkolonnen, ausgebildet. Bei Rückführung in einen Behälter des Bioreaktors T4 wird der Teilstrom C in die faulende Flüssigkeit eingeblasen. Ist ein Festbettreaktor T4c oder sind mehrere Festbettreaktoren T4c vorgesehen, so wird in solch einen Festbettreaktor T4c ausgefaulte Flüssigkeit von dem Überlauf des Bioreaktors T4 über die Leitung R6 zugeführt. Der Teilstrom C wird in einen Bodenbereich des Festbettreaktors T4c geführt. Dem aufsteigenden Teilstrom C rieselt in dem Festbettreaktor T4c oder in den Festbettreaktoren T4c die ausgefaulte Flüssigkeit von oben entgegen. Da der Festbettreaktor bzw. die mehreren Festbettreaktoren T4c bei sehr geringer Raum- und Faulschlammbelastung arbeitet bzw. arbeiten, werden die organischen Begleitstoffe im Teilstrom C stark abgebaut. Der derart gereinigte Teilstrom C wird dem Gasspeicher T8 zugeführt.

Falls infolge der Ausfaulung im Bioreaktor T4 das Auftreten von Begleitstoffen im Biogas wahrscheinlich ist, kann das rohe Biogas auch direkt, entweder vor oder nach dem Gasspeicher T8 über solch einen Festbettreaktor T4c geleitet werden. Hierfür dient eine von R9 abzweigende Leitung R9a, durch die das Biogas aus dem Biogasreaktor T4 in gleicher Weise wie der kohlendioxidreiche Teilstrom C durch den Festbettreaktor T4c geführt wird.

Für die Reinheit des Kohlendioxids ist es besonders vorteilhaft, wenn nach Durchlauf der zweiten Methan-CO₂-Trennstufe T12c und vor der Zuführung zum Kohlendioxidspeicher T13 oder einer direkten Einspeisung in ein Leitungsnetz eine weitere Aufkonzentrierung vorzugsweise im flüssigen Zustand durch Destillation, Strippen und/oder Aktivkohlefilter erfolgt.

Es soll schließlich auch daran hingewiesen werden, dass die anhand der Figur 9 beschriebene Gastrennungseinrichtung T12 und das im Zusammenhang mit Figur 9 betriebene Trennverfahren nicht nur bei einer in einem PSA-Verfahren betriebenen Gastrennungseinrichtung mit Vorteil eingesetzt werden kann, sondern beispielsweise auch im Zusammenhang mit Membranverfahren zur Gastrennung.

### Bezugszeichenliste

### Silierung

- T 1: Fahrsilo
- R 1: Rohr für CO₂
- V 1: Ventil im Silo
- M 1: Silofolie
- T 2: Sickersaftgrube
- Fl 1: Stutzen für Schlauchanschlüsse
- R 2: Schlauch für CO₂-Zugabe
- T 15: Tank für flüssiges CO₂
- K 5: CO₂-Gebläse

### Vormischung

- T 3: Vorgrube oder Seitenschacht
- Rü 1: Rührer mit Schneidrührwerkswirkung
- Mo 1: Motor am Schneidrührwerk
- P 1: Dickstoffpumpe
- V 2: Ventil an Dickstoffpumpe
- Fl 2: Obere Öffnung im Seitenschacht
- Fl 3: Untere Öffnung im Seitenschacht
- V 3: Verschluß an Fl 2
- P 2: Fördereinrichtung
- Fl 4: Deckel auf dem Seitenschacht
- T 3,1: Verlängerung des Seitenschachtes am Boden
- V 3,1: Verschluß an Fl 3
- R 3: Druckleitung zur Vorgrube

### Biogaserzeugung

- T 4: Biogasreaktor
- T 4a: Liegender oder stehender Fermenter
- T 4b: Stehender Fermenter
- T 4c: Füllkörperkolonne
- T 5: Kopfraum im Biogasreaktor
- Fl 5: Mannloch
- Fl 6: Stutzen für Überdrucksicherung im Biogasreaktor
- Fl 7: Stutzen für Unterdrucksicherung im Biogasreaktor
- Rü 2: Rührwerk im Biogasreaktor
- Rübl 1: Oberes Rührblatt am Rührwerk
- Rübl 2: Unteres Rührblatt am Rührwerk
- Mo 2: Motor am senkrechten Rührwerk im stehenden Fermenter
- Rü 3: Rührwerk im liegenden Fermenter
- Mo 3: Getriebemotor am Rührwerk im liegenden Fermenter
- P 3: Dosierpumpe für Chemikalienzugabe
- P 4: Entleerungspumpe für Biogasbehälter
- Iso 1: Thermische Isolierung am Biogasreaktor
- WT 1: Wärmetauscher Fußbodenheizung im Biogasreaktor
- Fl 9: Zuführöffnung
- Fl 10: Entleerungsöffnung
- Fl 11: Stutzen für Temperaturmessung
- Fl 12: Stutzen für Druckmessung
- Fl 13: Öffnung für Chemikalienzugabe
- V 5: Rückschlagklappe vor liegendem Fermenter T 4a
- V 6: Ventil vor außenliegendem Wärmetauscher
- V 7: Ventil in Wärmetauscherumgehungsleitung
- V 8: Ventil nach Dickstoffpumpe vor T 4a
- V 9: Ventil nach Dickstoffpumpe vor T 4b
- V 10: Ventil in Entleerungsleitung von T 4a zwischen T 4a und T 4b
- V 11: Ventil in Entleerungsleitung von T 4a vor V 10 in Saugleitung von Entleerungspumpe
- V 12: Ventil in Entleerungsleitung von T 4b in Saugleitung von Entleerungspumpe
- R 4: Druckleitung nach Dickstoffpumpe vor T 4a und T 4b
- R 5: Verbindungsleitung von Teilreaktor T 4a zu T 4b
- R 6: Überlaufleitung T 4a
- R 7: Überlaufleitung T 4b
- T 6: Nachgärtank
- Mo 4: Motor am Rührwerk im Nachgärtank
- Rü 4: Seitlich angebrachtes Rührwerk im Nachgärtank
- M 2: Gasspeichermembran über Nachgärtank
- WT 2: Außenliegender Wärmetauscher zur Erhitzung
- Iso 2: Thermische Isolierung am Wärmetauscher
- T 7: Haltetank für Hygienisierung
- Iso 3: Thermische Isolierung am Hygienisierungstank
- P 5: Heizungspumpe für Heißwasser aus Energiestation
- Mo 5: Motor an Förderpumpe nach Haltetank
- P 6: Förderpumpe nach Haltetank
- V 13: Ventil nach Haltetank
- V 14: Zweiwegeventil nach Förderpumpe am Haltetank
- WT 4: Heizmantel am liegenden Fermenter T 5,1
- V 15: Rückschlagklappe nach liegendem Behälter

### Gasspeicherung und -trennung

- T 8: Gasspeicher für rohes Biogas
- M 3: Folie im Biogasspeicher, roh und entschwefelt
- T 9: Feuerhemmende Umhüllung um Folienspeicher
- V 16: Kondensatablassventil am Biogasspeicher
- Fl 14: Anschluss für Kondensatablass am Biogasspeicher
- K 1: Gebläse für rohes Biogas
- V 17: Ventil in Rohgasleitung nach Gebläse
- R 8: Gasleitung nach Gebläse für rohes Biogas
- R 9: Gasleitung nach Biogasreaktor zum Gasspeicher für rohes Biogas
- T 10: Energiestation
- T 11: Gasspeicher für entschwefeltes Biogas
- K 2: Gebläse für entschwefeltes Biogas
- V 18: Ventil in Gasleitung für entschwefeltes Gas nach Gebläse
- R 10: Gasleitung nach Gebläse für entschwefeltes Biogas
- R 11: Gasleitung zur Energiestation
- R 12: Gasleitung zwischen den Zwischenräumen M 3,1
- T 12: Gastrennungseinrichtung
- T 12a: Entschwefelungsstufe
- T 12b: erste Methan-CO₂-Trennstufe
- T 12c: zweite Methan-CO₂-Trennstufe
- 20: Verzweigung
- T 13: Niederdruck CO₂-Speicher
- M 4: CO₂-Speichermembran für Niederdruckspeicher
- R 13: Rohrleitung für CO₂ nach Niederdruckspeicher
- K 3: CH₄-Kompressor 250 bar
- K 4: CH₄-Kompressor 50 bar
- Mo 8: Motor am CH₄-Kompressor 250 bar
- Mo 9: Motor am CH₄-Kompressor 50 bar
- T 14: CH₄-Tank 250 bar
- R 14: Gasleitung für Biomethan zum Erdgasnetz
- R 15: Gasleitung nach Entschwefelungsstufe zum Gasspeicher T 11
- K 5: CO₂-Gebläse zur direkten Entnahme
- Mo 10: Motor am CO₂-Gebläse
- K 6: CO₂-Kompressor zur Verflüssigung
- Mo 11: Motor am CO₂-Kompressor zur Verflüssigung
- T 15: Tank für flüssiges CO₂
- WT 5: Wärmetauscher in der Gastrennungseinrichtung
- V 19: Ventil in Abzweigung zum Zwischenraum in CO₂-Speichermembran (M 4)
- R 16: Leitung zum Zwischenraum in CO₂-Speichermembran
- T 16: Zwischenraum bei Gasspeicherfolie für CO₂

## Patentansprüche

1. Verfahren zur Erzeugung von Biogas, bei dem
a) eine Biomasse, vorzugsweise eine Mischung aus Energiepflanzen und Gülle und/oder organischem Abfall, mittels Mikroorganismen in einem Reaktorbehälter (T4a, T4b) fermentativ abgebaut wird,
b) Biomasse in einen außerhalb von dem Reaktorbehälter (T4a, T4b) angeordneten Seitenschacht (T3) eingegeben wird,
c) ausgefaulte Flüssigkeit aus dem Reaktorbehälter (T4a, T4b) auf die in dem Seitenschacht (T3) befindliche Biomasse geleitet
d) und die ausgefaulte Flüssigkeit zusammen mit der Biomasse durch eine untere Öffnung (Fl3) aus dem Seitenschacht (T3) in den Reaktorbehälter (T4a, T4b) gefördert wird.

2. Verfahren nach Anspruch 1, dadurch gekennzeichnet, dass die Biomasse und die ausgefaulte Flüssigkeit in dem Seitenschacht (T3) mittels eines Rührwerks (Rü1) vermischt werden und die Biomasse vorzugsweise zerkleinert wird.

3. Biogasreaktor, der Reaktor umfassend
a) wenigstens einen Reaktorbehälter (T4a, T4b) zur Erzeugung eines Biogases durch fermentativen Abbau einer Biomasse,
b) und einen Seitenschacht (T3) für eine Einbringung von Biomasse in den Reaktorbehälter (T4a, T4b),
c) wobei der Seitenschacht (T3) eine untere Öffnung (Fl3), durch welche Biomasse aus dem Seitenschacht (T3) in den fermentativen Abbau förderbar ist, und eine obere Öffnung (Fl2) aufweist, durch welche ausgefaulte Flüssigkeit aus dem Abbau in den Seitenschacht (T3) nachströmen kann,
**dadurch gekennzeichnet**, dass
d) der Seitenschacht (T3) außen an oder neben dem Reaktorbehälter (T4a, T4b) angeordnet und über die obere Öffnung (Fl2) und die untere Öffnung (Fl3) mit dem Reaktorbehälter (T4a, T4b) verbunden oder verbindbar ist.

4. Reaktor nach dem vorhergehenden Anspruch, dadurch gekennzeichnet, dass ein Verschluss (V3) zum Verschließen der oberen Öffnung (Fl2) und vorzugsweise auch ein Verschluss (V3) zum Verschließen der unteren Öffnung (Fl3) vorgesehen ist.

5. Reaktor nach einem der vorhergehenden Ansprüche, dadurch gekennzeichnet, dass die obere Öffnung (Fl2) auf solch einer Höhe angeordnet ist, dass Faulwasser aufgrund seines eigenen hydrostatischen Drucks aus dem Reaktorbehälter (T4a, T4b) in den Seitenschacht (T3) strömt und im Seitenschacht (T3) befindliche frische Biomasse zu der unteren Öffnung (Fl3) spült.

6. Reaktor nach einem der vorhergehenden Ansprüche, dadurch gekennzeichnet, dass aus dem Seitenschacht (T3) mittels einer vorzugsweise in dem Seitenschacht (T3) angeordneten Fördereinrichtung (P2) parallel zu einer Seitenwand des Reaktorbehälters (T4a, T4b)und vorzugsweise über eine abgerundete Ecke in den Reaktorbehälter (T4a, T4b) gefördert wird.

7. Reaktor nach einem der vorhergehenden Ansprüche, dadurch gekennzeichnet, dass der Seitenschacht (T3) von der unteren Öffnung (Fl3) aus in das Innere des Reaktorbehälters (T4a, T4b), vorzugsweise entlang einer Seitenwand des Reaktorbehälters (T4a, T4b), verlängert (T 3,1) ist.

8. Reaktor nach einem der vorhergehenden Ansprüche, dadurch gekennzeichnet, dass der Seitenschacht (T3) für ein Nachströmen der ausgefaulten Flüssigkeit über wenigstens zwei obere Öffnungen (Fl2), die auf unterschiedlichen Höhen angeordnet sind, mit dem Reaktorbehälter (T4a, T4b) verbunden oder verbindbar ist.

9. Reaktor nach einem der vorhergehenden Ansprüche, dadurch gekennzeichnet, dass ein Gasspeicher (T 8) für rohes Biogas vorgesehen und in einen Nachgärtank (T 6) des Reaktors (T 4) integriert ist, und dass eine Membran (M 2) auf dem Nachgärtank (T 6) schwimmt und ein von dem Reaktor (T4) zu dem Nachgärtank (T 6) geleitetes Biogas von unten durch die Flüssigkeitsoberfläche in den Gasspeicher (T8) unter der Membran (M 2) oder durch eine Behälterwand des Nachgärtanks (T 6) direkt in den Gasspeicher (T 8) unter der Membran (M 2) eintritt.

10. Reaktor nach einem der vorhergehenden Ansprüche, dadurch gekennzeichnet, dass ein Gasspeicher (T 11) für entschwefeltes Biogas von einer Membran (M 3) gegen Luftsauerstoff dicht verschlossen wird und vorzugsweise einen Schutzraum (M 3,1) bildet, der mit rohem und/oder entschwefeltem Biogas mit einem Überdruck von 1 bis 100 mbar und/oder einer Flüssigkeit gefüllt und vorzugsweise durchströmt ist.

11. Reaktor nach einem der vorhergehenden Ansprüche, dadurch gekennzeichnet, dass entschwefeltes Biogas aus einem Gasspeicher (T 11) abgesaugt und zu einer CO₂-CH4-Trennung einer Gastrennungseinrichtung (T 12) gefördert wird und die Gastrennungseinrichtung (T12) vorzugsweise an ein fest verlegtes Leitungsnetz für flüssiges oder gasförmiges CO₂ angeschlossen ist und aufbereitetes Kohlendioxid zur weiteren Verwertung vorzugsweise in das Leitungsnetz gefördert wird.
